# FASCICULE DE BREVET EUROPEEN

(11) **EP 1 931 377 B1**
(45) Date de publication et mention de la délivrance du brevet: **02.07.2014**
(21) Numéro de dépôt: 06808209.8
(22) Date de dépôt: 28.09.2006
(51) Int. Cl.: A61K 39/00, A61K 38/10, A61K 38/16, C07K 7/08, C07K 14/705, C07K 19/00, C12N 15/12, C12N 15/63, G01N 33/574, G01N 33/68, A61P 35/00

(54) **EPITOPES T CD4+ DE LA SURVIVINE ET LEURS APPLICATIONS**
CD4 + T SURVIVIN EPITOPE UND IHRE VERWENDUNGEN
CD4+ T SURVIVIN EPITOPES AND USES THEREOF

(30) Priorité: 30.09.2005 FR 0510013
(43) Date de publication de la demande: 18.06.2008
(73) Titulaire: Commissariat à l'Énergie Atomique et aux Énergies Alternatives, 75015 Paris (FR)
(72) Inventeur: WANG, XiaoFei, 75002 Paris (FR); MUNIER, Gaétan, 91400 Orsay (FR); MORATILLE, Sandra, 91700 Sainte Geneviève des Bois (FR); NUYTTENS, Hélène, 75014 Paris (FR); MAILLERE, Bernard, 78000 Versailles (FR)
(74) Mandataire: Leblois-Préhaud, Hélène Marthe Georgette
(86) Numéro de dépôt international: PCT/FR2006/002196
(87) Numéro de publication internationale: WO 2007/036638

(56) Documents cités:
- WO-A-00/03693
- WO-A-03/040299
- WO-A-2004/055183
- WO-A-2004/067023
- WO-A1-00/03693
- FORTUGNO P ET AL: "Survivin exists in immunochemically distinct subcellular pools and is involved in spindle microtubule function" JOURNAL OF CELL SCIENCE, CAMBRIDGE UNIVERSITY PRESS, LONDON, GB, vol. 115, no. 3, 1 février 2002 (2002-02-01), pages 575-585, XP002283857 ISSN: 0021-9533
- CASATI CHIARA ET AL: "The apoptosis inhibitor protein survivin induces tumor-specific CD8+ and CD4+ T cells in colorectal cancer patients." CANCER RESEARCH, vol. 63, no. 15, 1 août 2003 (2003-08-01), pages 4507-4515, XP002385073 ISSN: 0008-5472
- SCHMITZ M ET AL: "Generation of survivin-specific CD8+ T effector cells by dendritic cells pulsed with protein or selected peptides", CANCER RESEARCH, AMERICAN ASSOCIATION FOR CANCER REREARCH, US, vol. 60, no. 17, 1 September 2000 (2000-09-01), pages 4845-4849, XP002283855, ISSN: 0008-5472
- PIESCHE M: "Identifikation und immunologische Characterisierung von MHC-Klasse-II Peptidepitopen in humanen leukÃ mie- und lymphom-assoziierten Antigenen", DISSERTATION UNIVERSITAET GOETTINGEN,, 1 May 2006 (2006-05-01), pages 1-3,67,86, XP003025356,

### SEQUENCE LISTING

<110> Commissariat à l'Energie Atomique
   WANG XiaoFei
   MUNIER Gaetan
   MORATILLE Sandra
   NUYTTENS Hélène
   MAILLERE Bernard
<120> Epitopes T CD4+ de la survivine et leurs applications
<130> F263PCT145
<160> 41
<170> PatentIn version 3.3
<210> 1
   <211> 15
   <212> PRT
   <213> Homo sapiens
<400> 1
   Met Gly Ala Pro Thr Leu Pro Pro Ala Trp Gln Pro Phe Leu Lys
   1               5                   10                  15
<210> 2
   <211> 15
   <212> PRT
   <213> Homo sapiens
<400> 2
   Pro Thr Leu Pro Pro Ala Trp Gln Pro Phe Leu Lys Asp His Arg
   1               5                   10                  15
<210> 3
   <211> 15
   <212> PRT
   <213> Homo sapiens
<400> 3
   Pro Ala Trp Gln Pro Phe Leu Lys Asp His Arg Ile Ser Thr Phe
   1               5                   10                  15
<210> 4
   <211> 15
   <212> PRT
   <213> Homo sapiens
<400> 4
   Gln Pro Phe Leu Lys Asp His Arg Ile Ser Thr Phe Lys Asn Trp
   1               5                   10                  15
<210> 5
   <211> 15
   <212> PRT
   <213> Homo sapiens
<400> 5
   His Arg Ile Ser Thr Phe Lys Asn Trp Pro Phe Leu Glu Gly Cys
   1               5                   10                  15
<210> 6
   <211> 15
   <212> PRT
   <213> Homo sapiens
<400> 6
   Ile Ser Thr Phe Lys Asn Trp Pro Phe Leu Glu Gly Cys Ala Cys
   1               5                   10                  15
<210> 7
   <211> 15
   <212> PRT
   <213> Homo sapiens
<400> 7
   Ser Thr Phe Lys Asn Trp Pro Phe Leu Glu Gly Cys Ala Cys Thr
   1               5                   10                  15
<210> 8
   <211> 15
   <212> PRT
   <213> Homo sapiens
<400> 8
   Lys Asn Trp Pro Phe Leu Glu Gly Cys Ala Cys Thr Pro Glu Arg
   1               5                   10                  15
<210> 9
   <211> 15
   <212> PRT
   <213> Homo sapiens
<400> 9
   Glu Arg Met Ala Glu Ala Gly Phe Ile His Cys Pro Thr Glu Asn
   1               5                   10                  15
<210> 10
   <211> 15
   <212> PRT
   <213> Homo sapiens
<400> 10
   Ala Gly Phe Ile His Cys Pro Thr Glu Asn Glu Pro Asp Leu Ala
   1               5                   10                  15
<210> 11
   <211> 15
   <212> PRT
   <213> Homo sapiens
<400> 11
   Pro Asp Leu Ala Gln Cys Phe Phe Cys Phe Lys Glu Leu Glu Gly
   1               5                   10                  15
<210> 12
   <211> 15
   <212> PRT
   <213> Homo sapiens
<400> 12
   Gln Cys Phe Phe Cys Phe Lys Glu Leu Glu Gly Trp Glu Pro Asp
   1 5 10 15
<210> 13
   <211> 15
   <212> PRT
   <213> Homo sapiens
<400> 13
   Phe Cys Phe Lys Glu Leu Glu Gly Trp Glu Pro Asp Asp Asp Pro
   1 5 10 15
<210> 14
   <211> 15
   <212> PRT
   <213> Homo sapiens
<400> 14
   Lys Glu Leu Glu Gly Trp Glu Pro Asp Asp Asp Pro Ile Glu Glu
   1 5 10 15
<210> 15
   <211> 15
   <212> PRT
   <213> Homo sapiens
<400> 15
   Glu Gly Trp Glu Pro Asp Asp Asp Pro Ile Glu Glu His Lys Lys
   1 5 10 15
<210> 16
   <211> 15
   <212> PRT
   <213> Homo sapiens
<400> 16
   Asp Pro Ile Glu Glu His Lys Lys His Ser Ser Gly Cys Ala Phe
   1 5 10 15
<210> 17
   <211> 15
   <212> PRT
   <213> Homo sapiens
<400> 17
   Cys Ala Phe Leu Ser Val Lys Lys Gln Phe Glu Glu Leu Thr Leu
   1 5 10 15
<210> 18
   <211> 15
   <212> PRT
   <213> Homo sapiens
<400> 18
   Leu Ser Val Lys Lys Gln Phe Glu Glu Leu Thr Leu Gly Glu Phe
   1               5                   10                  15
<210> 19
   <211> 15
   <212> PRT
   <213> Homo sapiens
<400> 19
   Lys Lys Gln Phe Glu Glu Leu Thr Leu Gly Glu Phe Leu Lys Leu
   1               5                   10                  15
<210> 20
   <211> 15
   <212> PRT
   <213> Homo sapiens
<400> 20
   Lys Gln Phe Glu Glu Leu Thr Leu Gly Glu Phe Leu Lys Leu Asp
   1               5                   10                  15
<210> 21
   <211> 15
   <212> PRT
   <213> Homo sapiens
<400> 21
   Phe Glu Glu Leu Thr Leu Gly Glu Phe Leu Lys Leu Asp Arg Glu
   1               5                   10                  15
<210> 22
   <211> 15
   <212> PRT
   <213> Homo sapiens
<400> 22
   Glu Glu Leu Thr Leu Gly Glu Phe Leu Lys Leu Asp Arg Glu Arg
   1               5                   10                  15
<210> 23
   <211> 15
   <212> PRT
   <213> Homo sapiens
<400> 23
   Leu Thr Leu Gly Glu Phe Leu Lys Leu Asp Arg Glu Arg Ala Lys
   1               5                   10                  15
<210> 24
   <211> 15
   <212> PRT
   <213> Homo sapiens
<400> 24
   Gly Glu Phe Leu Lys Leu Asp Arg Glu Arg Ala Lys Asn Lys Ile
   1               5                   10                  15
<210> 25
   <211> 15
   <212> PRT
   <213> Homo sapiens
<400> 25
   Leu Lys Leu Asp Arg Glu Arg Ala Lys Asn Lys Ile Ala Lys Glu
   1               5                   10                  15
<210> 26
   <211> 15
   <212> PRT
   <213> Homo sapiens
<400> 26
   Asn Lys Ile Ala Lys Glu Thr Asn Asn Lys Lys Lys Glu Phe Glu
   1               5                   10                  15
<210> 27
   <211> 15
   <212> PRT
   <213> Homo sapiens
<400> 27
   Lys Glu Phe Glu Glu Thr Ala Lys Lys Val Arg Arg Ala Ile Glu
   1               5                   10                  15
<210> 28
   <211> 15
   <212> PRT
   <213> Homo sapiens
<400> 28
   Ala Lys Lys Val Arg Arg Ala Ile Glu Gln Leu Ala Ala Met Asp
   1               5                   10                  15
<210> 29
   <211> 13
   <212> PRT
   <213> ARTIFICIAL SEQUENCE
<220>
   <223> HA 306-318
<400> 29
   Pro Lys Tyr Val Lys Gln Asn Thr Leu Lys Leu Ala Thr
   1               5                   10
<210> 30
   <211> 15
   <212> PRT
   <213> artificial sequence
<220>
   <223> A3 152-166
<400> 30
   Glu Ala Glu Gln Leu Arg Ala Tyr Leu Asp Gly Thr Gly Val Glu
   1               5                   10                  15
<210> 31
   <211> 15
   <212> PRT
   <213> artificial sequence
<220>
   <223> MT 2-16
<400> 31
   Ala Lys Thr Ile Ala Tyr Asp Glu Glu Ala Arg Arg Gly Leu Glu
   1               5                   10                  15
<210> 32
   <211> 16
   <212> PRT
   <213> artificial sequence
<220>
   <223> B1 21-36
<400> 32
   Thr Glu Arg Val Arg Leu Val Thr Arg His Ile Tyr Asn Arg Glu Glu
   1 5 10 15
<210> 33
   <211> 13
   <212> PRT
   <213> artificial sequence
<220>
   <223> YKL
<400> 33
   Ala Ala Tyr Ala Ala Ala Lys Ala Ala Ala Leu Ala Ala
   1 5 10
<210> 34
   <211> 20
   <212> PRT
   <213> artificial sequence
<220>
   <223> LOL 191-210
<400> 34
   Glu Ser Trp Gly Ala Val Trp Arg Ile Asp Thr Pro Asp Lys Leu Thr
   1 5 10 15
   Gly Pro Phe Thr
   20
<210> 35
   <211> 14
   <212> PRT
   <213> artificial sequence
<220>
   <223> E2/E168
<400> 35
   Ala Gly Asp Leu Leu Ala Ile Glu Thr Asp Lys Ala Thr Ile
   1 5 10
<210> 36
   <211> 17
   <212> PRT
   <213> artificial sequence
<220>
   <223> oxy 271-287
<400> 36
<210> 37
   <211> 9
   <212> PRT
   <213> Homo sapiens
<400> 37
   Leu Thr Leu Gly Glu Phe Leu Lys Leu
   1 5
<210> 38
   <211> 10
   <212> PRT
   <213> Homo sapiens
<400> 38
   Glu Leu Thr Leu Gly Glu Phe Leu Lys Leu
   1 5 10
<210> 39
   <211> 9
   <212> PRT
   <213> Homo sapiens
<400> 39
   Ala Tyr Ala Cys Asn Thr Ser Thr Leu
   1 5
<210> 40
   <211> 24
   <212> PRT
   <213> Homo sapiens
<400> 40
   Ile Gly Pro Gly Thr Val Ala Tyr Ala Cys Asn Thr Ser Thr Leu Gly
   1 5 10 15
   Gly Arg Gly Gly Arg Ile Thr Arg
   20
<210> 41
   <211> 11
   <212> PRT
   <213> artificial séquence
<220>
   <223> PADRE
<220>
   <221> misc_feature
   <222> (2)..(2)
   <223> Xaa can be any naturally occurring amino acid
<400> 41
   Lys Xaa Val Ala Ala Trp Thr Leu Lys Ala Ala
   1               5                   10

## Description

La présente Invention est relative à des peptides représentant des épitopes T CD4+ de la survivine aptes à être présentés par des molécules HLA II prépondérantes, notamment dans la population caucasienne, et à leurs applications vaccinales et diagnostiques.

Les antigènes tumoraux regroupent un ensemble de protéines exprimées par les cellules tumorales et que les cellules saines n'expriment peu ou pas, ou qui ne sont retrouvées que dans quelques types cellulaires. Ces antigènes sont classés en cinq catégories en fonction de leur profil d'expression : (1) les antigènes spécifiques du patient, résultant de mutations ponctuelles liées à la tumorigénèse (MUM-1, CDK4, béta-caténine, HLA-A2, BCR-ABL, CASP-8), (2) les antigènes spécifiques de tumeurs ou antigènes CT (*Cancer Testis*), exprimés dans de nombreuses tumeurs et quelques tissus sains dépourvus de molécules HLA conventionnelles (testicules, placenta, ovaires ; antigènes MAGE, BAGE, GAGE, RAGE, NY-ESO1), (3) les antigènes de différenciation qui s'expriment soit au cours de l'embryogénèse, soit dans des types cellulaires bien particuliers (tyrosinases, gp-100, Melan-A/mart-1, thyroglobuline, alpha-foetoprotéine, CEA), (4) les antigènes surexprimés par les tumeurs (survivine, gp75, PSA, HER-2/neu, p53, télomérase) et (5) les antigènes viraux (EBV, HPV).

Toutes ces protéines qui peuvent être reconnues par des lymphocytes T CD4+ et CD8+ et servir à l'induction d'une immunité anti-tumorale représentent des cibles pour la vaccination anti-tumorale (Demande Internationale PCT WO 2004/055183).

Les cibles qui ont été particulièrement bien étudiées sont principalement des antigènes spécifiques de tumeurs (MAGE, NY-ESO-1). Toutefois, ces antigènes peuvent être des cibles inefficaces pour la vaccination antitumorale, dans la mesure où la plupart ne sont pas essentiels à la survie des cellules tumorales qui peuvent échapper à la surveillance immunologique en diminuant ou en réprimant l'expression de ces antigènes.

La survivine (16,5 kDa), également dénommée BIRC5 (*Baculoviral IAP repeat-containiing protein* 5), IAP4 (*Inhibitor of Apoptosis Protein 4)* ou API4 (*Apoptosis Inhibitor 4*) est le membre le plus petit de la famille des inhibiteurs de l'apoptose (IAP); elle contient un motif à doigt de zinc de 70 acides aminés (domaine BIR pour *Baculovirus Inhibitor of Apoptosis Protein Repeat*), caractéristique de tous les membres de la famille IAP et une extrémité C-terminale comprenant une hélice alpha *coiled-coil.* Le gène codant pour la survivine est localisé sur le chromosome 17 humain (17q25) ou 11 murin (11 E2) ; l'épissage alternatif d'un pré-mRNA comprenant les exons 1, 2, 2B, 3 et 4 produit trois transcrits : (1) un transcrit comprenant les exons 1, 2, 3 et 4, codant pour l'isoforme alpha de la survivine (142 acides aminés GenBank AAC51660 ou SwissProt 015392), (2) un transcrit comprenant les exons 1, 2, 2B, 3 et 4, codant pour l'isoforme béta ou survivine-2B (165 acides aminés) qui résulte de l'insertion de la séquence IGPGTVAYACNTSTLGGRGGRITR (23 acides aminés, SEQ ID NO : 40), entre les positions 74 et 75 de la séquence de l'isoforme alpha et (3) un transcrit comprenant les exons 1, 2, et 4, codant pour la survivine ΔEx-3.

La survivine est surexprimée dans la majorité des tumeurs, notamment dans celles associées aux cancers du sein, foie, colon, poumon, ovaire, utérus, oesophage, estomac, pancréas, foie et prostate, à la maladie de Hodgkin, au syndrome myélodisplasique avec anémie réfractaire, aux mélanomes, lymphomes non hodgkiniens, leucémies, neuroblastomes, phéochromocytomes, sarcomes des tissus mous et tumeurs cérébrales. La surexpression de cette protéine non-redondante entraîne une insensibilité à l'apoptose et favorise la division cellulaire. En outre, cette surexpression de la survivine est essentielle à la survie des cellules tumorales ; l'extinction de l'expression de la survivine dans des lignées de cellules tumorales humaines par des méthodes antisens, ou des mutants dominants négatifs de la survivine résulte en l'arrêt de la division cellulaire et l'apoptose (Yang et al., P.N.A.S., 2004, 101, 15100-15105 ; Altieri et al., Oncogene, 2003, 22, 8581-8589).

Cet antigène tumoral est un bon indicateur du pronostic du cancer un taux élevé de survivine (ARNm) est corrélé avec un pronostic défavorable (Takeuchi et al., Int. J. Cancer, 2005 ; Rodel et al., Cancer Rés., 2005, 65, 4881-4887 ; Muzio et al., CancerLett., 2005, 225, 27-33 ; Kim et al., Cancer Lett., 2005, 224, 253-261).

En revanche, la survivine est pratiquement indétectable dans les tissus normaux différenciés. Elle possède une durée de vie très courte et est présente uniquement pendant la mitose où elle participe à la formation du complexe CPPs (*Chromosomal Passenger Proteins*) qui comprend la kinase Aurora B, la protéine INCENP (*Inner Centromere Protein*) et TD60 (*Telophase Disk Antigen*), et est également impliquée dans l'inhibition de l'apoptose en réprimant l'activité des caspases 3, 7, 9 (Schimmer, A.D., Cancer Research, 2004, 64, 7183-7190 ; Fortugno et al. J. Cell. Science, 2002, 115, 575-585).

L'étude de la réponse immune dirigée contre la survivine, chez des individus atteints de cancer, indique que cet antigène tumoral peut induire une réponse cellulaire impliquant des lymphocytes T CD4+ et T CD8+ spécifiques (Andersen et al., Cancer Research, 2001, 61, 869-872 et 5964-5968 ; Schmidt et al., Blood, 2003, 102, 571-576; Casati et al., Cancer Research, 2003, 63, 4507-4515 ; Ichiki et al., Lung Cancer, 2005, 48, 281-289).

Casati et al., Cancer Research, 63, 4507-4515, 2003 dévoilent que des patients atteints de cancer colorectal possèdent des cellules T-CD4+ spécifiques de la survivine.Des anticorps spécifiques sont également détectés ; le taux élevé d'anticorps anti-survivine détecté dans des cas de cancer de pronostic défavorable indique toutefois que la réponse humorale ne semble pas impliquée dans le rejet des tumeurs exprimant la survivine *(Ichiki et al.,* précité).

En conséquence, la survivine représente une cible particulièrement intéressante pour la vaccination anti-tumorale du fait qu'elle induit une réponse T CD4+ et CD8+ spécifique des tumeurs, qu'elle est exprimée dans la plupart des tumeurs et que son expression est essentielle à la survie des tumeurs. En effet, l'utilisation d'un antigène essentiel à la survie des tumeurs, comme cible pour la vaccination anti-tumorale permet d'éviter les problèmes d'échappement des tumeurs à la reconnaissance par le système immunitaire. En outre, la plupart des cancers peuvent être traités avec un seul vaccin, du fait que la survivine est exprimée dans la majorité des tumeurs.

Ainsi, il a été proposé d'utiliser la survivine (protéine recombinante), un vecteur d'expression de cet antigène ou des cellules dendritiques transfectées par un tel vecteur d'expression, comme vaccin anti-tumoral (Demande Internationale PCT WO 00/03693; Pisarev et al., Clin., Cancer Res., 2003, 9, 6523-6533; Siegel et al., J. Immunol., 2003, 170, 5391-5397; Schaft et al., J. Immunol., 2005, 174, 3087-3097). Toutefois, pour induire à la fois une réponse T CD4+ et une réponse T CD8+ efficaces contre un antigène, il est préférable d'utiliser des peptides représentant les épitopes T CD4+ et T CD8+ de cet antigène, plutôt que la protéine entière.

Des épitopes T CD8+ de la survivine restreints aux molécules HLA-A1, A2, A3, A11, A24, B7, B8, B15, B35 du complexe majeur d'histocompatibilité de classe I, (molécules HLA I ou *Human Leucocyte Antigen class* I) ont été identifiés (peptides 95-104 et 96-104 restreints à HLA-A2 (Andersen et al., Cancer Res., 2001, 61, 869-872 ; Schmitz et al., Cancer Res., 2000, 60, 4845-4849 ; Schmidt et al., 2003, précité ; http://www.cancerimmunity.org/peptidedatabase/tumorspecific.htm ; Demande Internationale PCT WO 2004/067023) ; peptide survivine 2B 80-88 restreint à HLA-A24 (Hirohashi et al., Clin. Cancer Res., 2002, 8, 1731-1739) ; Recker et al., Int. J. Cancer 2004, 108, 937-941 et Cancer Biol. Ther., 2004, 3, 173-179 ; Bachinsky et al., Cancer Immun., 2005, 5, 6). Des cellules dendritiques autologues chargées avec le peptide 96-104 ont été utilisées pour vacciner des patients atteints de mélanome (Andersen et al., Vaccine , 2005, 23, 884-889).

Toutefois, l'immunisation avec des épitopes T CD8+ seuls, induit des lymphocytes T cytotoxiques spécifiques de l'antigène tumoral, avec une fréquence très faible (de l'ordre de 10⁻⁴ à 10⁻⁷ des cellules CD8+ ; Zhang et al., Eur. J. Immunol., 2005, 35, 776-785). En effet, l'induction de lymphocytes T cytotoxiques (CTL) est dépendante de l'activation des lymphocytes T CD4+ qui interviennent en particulier dans le recrutement et le maintien des CTL. Il a été montré que les lymphocytes T CD4+ jouent un rôle essentiel dans le contrôle des tumeurs. Par l'intermédiaire de contacts cellulaires et la sécrétion de nombreuses cytokines, les lymphocytes T CD4+ induisent l'activation des cellules présentatrices d'antigène (APC pour *Antigen Presenting Cell)* qui à leur tour recrutent des lymphocytes T CD8+ spécifiques des tumeurs. Ils interviennent également dans la maturation des cellules effectrices que sont les lymphocytes T cytotoxiques. En outre, les résultats observés chez des souris n'exprimant pas de molécules du complexe majeur d'histocompatibilité de classe I, indiquent également que les lymphocytes T CD4+ exercent un contrôle des tumeurs via des mécanismes indépendants des CTL probablement via l'activation de macrophages. Enfin, les lymphocytes T CD4+ peuvent être eux-mêmes cytotoxiques.

Les lymphocytes T CD4⁺ reconnaissent les peptides de tumeurs que leur présentent les molécules du complexe majeur d'histocompatibilité de classe II ; chez l'homme, elles sont dénommées molécules HLA II pour *Human Leucocyte Antigen class II.* La reconnaissance peut avoir lieu de manière directe (c'est-à-dire que la tumeur présente elle-même ces peptides aux lymphocytes T) mais il est vraisemblable que la voie principale d'activation se fasse par l'intermédiaire des cellules dendritiques. Ces cellules sont en effet les principales cellules présentatrices de l'antigène aptes à recruter *in vivo* les lymphocytes T naïfs. Ces peptides anti-géniques, appelés épitopes T, résultent de la dégradation protéolytique des antigènes par les cellules présentatrices d'antigène. Ils ont des longueurs variables, généralement de 13 à 25 acides aminés et possèdent une séquence qui les rend capables de se lier aux molécules HLA II. Il est bien connu qu'au même titre que l'antigène natif, un peptide comprenant un épitope T CD4+ est capable de stimuler *in vitro* des cellules T CD4+ qui lui sont spécifiques ou de les recruter *in vivo.* Il est donc suffisant pour induire une réponse T CD4+.

Ces observations sont en faveur de l'utilisation de tels peptides antigéniques spécifiques de la survivine, et capables de stimuler une forte réponse T CD4+, pour la préparation d'une composition vaccinale anti-tumorale chez l'Homme, notamment en association avec des épitopes T CD8+ spécifiques du même antigène.

En outre, de tels peptides qui sont reconnus par des lymphocytes T CD4+ spécifiques d'un antigène tumoral sont également utiles comme réactifs dans un test de diagnostic, d'évaluation du pronostic ou de suivi du traitement d'un cancer chez l'Homme, reposant sur la détection desdites cellules T CD4+, soit de manière directe, notamment par cytométrie de flux en présence de multimères de complexes molécules de classe II/peptide, soit de manière indirecte, notamment par un test de prolifération lymphocytaire, ou bien un dosage d'anticorps ou de cytokines.

Toutefois, aucun épitope T CD4+ de la survivine n'a été identifié. En effet, un des problèmes majeurs qui limite l'utilisation de ces peptides comme antigène est l'identification des épitopes T CD4+, étant donné que leur séquence varie d'un individu à l'autre en raison du polymorphisme des molécules HLA II. Les molécules HLA II sont des hétérodimères constitués d'une chaîne alpha (α) et d'une chaîne béta (β) polymorphes. Il existe quatre types de molécules HLA II par individu (2 HLA-DR, 1 HLA-DQ et 1 HLA-DP), dénommées en fonction de l'allèle codant la chaîne béta qui est la plus polymorphe. La molécule HLA-DR est très polymorphe. En effet, alors que sa chaîne alpha ne compte que 3 allèles, la chaîne béta (β) codée par le gène DRB1, qui est la plus exprimée, compte à ce jour 458 allèles. Pour les molécules HLA-DQ et HLA-DP, les deux chaînes (α et β) qui les constituent sont polymorphes mais elles présentent moins d'allèles. On a dénombré 28 allèles DQA1 (chaîne α de HLA-DQ), 60 allèles DQB1 (chaîne β de HLA-DQ), 22 allèles DPA1 (chaîne α de HLA-DP) et 116 allèles DPB1 (chaîne β de HLA-DP). Cependant, la combinaison entre les deux chaînes α et β codées par ces allèles donne naissance à de nombreuses molécules HLA-DQ et HLA-DP.

Du fait de ce polymorphisme, ces isoformes possèdent des propriétés de liaison différentes entre elles, ce qui implique qu'elles peuvent lier des peptides différents d'un même antigène. Ainsi, le mode de liaison des peptides aux molécules HLA II est plus complexe que celui des molécules HLA I et il n'existe pas de programmes fiables de prédiction des peptides capables de se lier aux molécules HLA II. En effet, les molécules HLA possèdent une structure tridimensionnelle très proche mais le site de fixation des molécules HLA II est une gorge ouverte aux deux extrémités. Pour cette raison, les molécules HLA II acceptent des peptides restreints aux molécules HLA II de taille variable, généralement comprise entre 13 et 25 acides aminés. Cette gorge est caractérisée par cinq poches de spécificité (P1, P4, P6, P7 et P9 correspondant aux positions des résidus du peptide qu'elles accommodent) qui abritent des résidus polymorphes. Comme les différences de séquences entre les différentes molécules HLA II sont essentiellement localisées dans les poches du site de fixation des peptides, elles interviennent directement dans le répertoire de peptides qui se lient à chaque molécule.

Ainsi, chaque individu reconnaît dans un antigène un ensemble de peptides dont la nature dépend des molécules HLA II qui le caractérisent. Comme il existe un grand nombre d'allèles HLA II, il existe donc dans une séquence donnée un répertoire important d'épitopes T de séquences très différentes, chacun spécifique d'un allèle différent. Un peptide capable de stimuler une réponse T CD4+ spécifique d'un antigène tumoral chez quelques individus peut donc être inactif chez la majorité des autres individus, parce que ces derniers ne reconnaissent pas l'antigène tumoral par les mêmes épitopes.

Toutefois, les molécules HLA II ne semblent pas être utilisées de manière uniforme, pour la reconnaissance des tumeurs. En effet, les épitopes T issus d'antigènes tumoraux sont pour la plupart restreints aux molécules HLA-DR, suggé rant que les molécules HLA-DQ ne sont apparemment que faiblement utilisées pour la reconnaissance des tumeurs.

En outre, les allèles HLA II ne sont pas répartis dans le monde de manière uniforme. Ainsi dix molécules HLA-DR sont prépondérantes en Europe et aux Etats-Unis et couvrent l'essentiel de la population ; il s'agit des molécules dont la chaîne béta est codée par les sept allèles du locus HLA-DRB1: *0101, *0301, *0401, *0701, *1101, *1301, *1501, et les allèles DRB3*0101, DRB4*0101 et DRB5*0101 (Tableau I).

**Tableau I : Fréquence génique (allélique*)/phénotypique d'HLA II**

| **Allèles** | **Europe** | | **USA** | | **Afrique** | **Asie** | |
|---|---|---|---|---|---|---|---|
| | **France** | **Allemagne** | **Caucasien** | **Afro-américain** | **Sénégal** | **Inde** | **Japon** |
| **DRB1*0101** | 9,3/17,7 | 6,7/13 | 7,3/14,1 | 1,9/3,8 | 0,6/1,2 | 3,8/7,5 | 4,9/9,6 |
| **DRB1*0401** | 5,6/10,9 | 8,1/15,5 | 6,7/13 | 1,5/3,0 | 0/0 | 0,9/1,8 | 0/0 |
| **DRB1*1101** | 9,2/17,6 | 9,2/17,6 | 4,4/8,6 | 8,2/15,7 | 9,3/17,7 | 0,9/1,8 | 2/4 |
| **DRB1*0701** | 14,0/26 | 12,3/23,1 | 14,4/26,7 | 9,8/18,6 | 7,8/15 | 13/24,3 | 0,6/1,2 |
| **DRB1*0301** | 10,9/20,6 | 9,4/17,9 | 9,5/18,1 | 7/13,5 | 10,2/19,4 | 5,3/10,3 | 0,4/0,8 |
| **DRB1*1301** | 6,0/11,6 | 4,5/8,8 | 5,1/9,9 | 4,2/8,2 | 4,7/9,2 | 6,3/12,2 | 0,7/1,4 |
| **DRB1*1501** | 8,0/14,4 | 7,8/15 | 10,3/19,5 | 8,6/16,5 | 0/0 | 12,1/22,7 | 9,1/17,4 |
| **TOTAL** | 63,0/86,3 | 58,0/82,4 | 57,7/82,1 | 41,2/65,4 | 32,/54,66 | 42,3/66,7 | 17,7/32,3 |
| | | | | | | | |
| **DRB5*0101** | 7,9/15,2 | 4,6/9 | 2,4/4,7 | 10,4/19,7 | 0/0 | 0/0 | 5,6/10,9 |
| **DRB3*0101** | 9,2/17,6 | 9,8/18,6 | 10,4/19,7 | 15,1/27,9 | 6,9/13,3 | 4,9/9,6 | 6,5/12,6 |
| **DRB4*0101** | 28,0/48,2 | 21,1/37,7 | 19,8/35,7 | 16,5/30,3 | 6,9/13,3 | 24,8/43,4 | 28,9/49,4 |
| **TOTAL** | 45,1/69,9 | 35,5/58,4 | 32,6/54,6 | 42,0/66,4 | 13,8/25,7 | 29,7/50,6 | 41,0/65,2 |
| | | | | | | | |
| **DPB1*0101** | 7,1/13,7 | 2,2/4,4 | 3,2/6,3 | 27,7/47,7 | 18,2/33,1 | nd | 0,1/0,2 |
| **DPB1*0201** | 11,9//22,4 | 8,5/16,3 | 9,8/18,6 | 12,9/24,1 | 13,8/25,7 | nd | 20,6/37 |
| **DPB1*0301** | 17,0/31,1 | 3,8/7,5 | 7,4/14,3 | 3,3/6,5 | 3,8/7,5 | nd | 3/5,9 |
| **DPB1_{*}0401** | 40,0/64 | 38,1/61,7 | 25,1/43,9 | 11/20,8 | 4,8/9,4 | nd | 4,7/9,2 |
| **DPB1*0402** | 11,0/20,8 | 15,4/28,4 | 12,6/23,6 | 9/17,2 | 25,5/44,5 | nd | 36,8/60,1 |
| **TOTAL** | 87,0/98,3 | 68,0/89,8 | 58,1/82,4 | 63,9/87,0 | 66,1/88,5 | nd | 65,2/87,9 |
| **Total** | 51/76 | 53,5/78,4 | 37,7/61,2 | 20/36,0 | 30,3/51,4 | | 41,5/65,8 |
| **DP401+402** | | | | | | nd | |

| | | | | | | | |
|---|---|---|---|---|---|---|---|
| * les molécules HLA II prépondérantes (fréquence génique> 5 %) sont indiquées en gras | | | | | | | |

Par exemple, dans la population française qui est une population caractéristique de la population caucasienne (USA, Europe), seuls les 7 allèles du locus DRB1 ont une fréquence allélique supérieure à 5 %; ces 7 allèles DRB1 représentent à eux seuls 63 % de la population. Ces mêmes allèles DRB1 sont les allèles HLA-DR les plus abondants dans les autres populations caucasiennes. Leur fréquence varie entre 53 % (en Espagne) et 82 % (au Danemark). Pour les États-Unis et le Canada, ils représentent respectivement 58 et 55 % des allèles de la population.

En outre certains des allèles DRB1, les plus fréquents dans la population caucasienne sont également fréquents dans la population africaine (DRB1*0301 , *0701 et *1101), indienne (DRB1*0301 , *0701, *1301 et *1501) et japonaise (DRB1*1501)

Les molécules HLA-DRB3, -DRB4 et DRB5 qui sont des molécules HLA-DR dont la chaîne β n'est pas codée par le gène DRB1 sont également prépondérantes en Europe, aux USA et au Japon, car elles sont moins polymorphes que les molécules DRB 1 (Tableau I). Par exemple, en France, leur fréquence allélique est de 9,2 % pour DRB3*0101, 28 % pour DRB4*0101 et 7,9 % pour DRB5*0101. Elles couvrent donc à elles seules 45 % de la fréquence allélique en France.

De plus, les motifs de liaison de certaines molécules HLA-DR sont identiques si bien qu'un peptide peut se lier à plusieurs molécules HLA-DR. En outre, il n'est pas rare que les épitopes T CD4+ soient imbriqués les uns dans les autres si bien qu'une séquence peptidique peut contenir plusieurs épitopes T CD4+.

Enfin, deux molécules HLA-DP4 (DP401 et DP402) couvrent à elles seules la majorité de la fréquence allélique d'HLA-DP dans la population caucasienne (de l'ordre de 50 % en Europe et 80 % en Amérique du Nord) et sont également présentes à des fréquences non négligeables dans les autres populations (fréquence allélique de l'ordre de 60 % en Amérique du Sud, 60 % en Inde, 30 % en Afrique et 40 % au Japon ; Tableau I). Chacune de ces molécules DP4 comprend une chaîne alpha codée, soit par l'allèle DPA1*0103 qui est le plus fréquent (78,2 %), soit par l'allèle DPA1*0201 (20,2 %) et une chaîne béta codée par l'allèle DPB1*0401 (molécule HLA-DP401) ou DPB1*0402 (molécule HLA-DP402).

Comme les molécules HLA-DR, les molécules HLA-DP4 sont parfaitement aptes à présenter des peptides aux lymphocytes T dans la mesure où de nombreux clones T restreints à HLA-DP4 ont été isolés à partir d'antigènes très variés tels que la toxine tétanique, le virus de l'hépatite B et l'allergène majeur de *Dermatophagoïdes pteronissimus.*

Les peptides, qui lient les molécules HLA II codées par les allèles les plus fréquents dans la population (molécules HLA II prépondérantes), incluent donc les épitopes T de la majorité de la population.

Les inventeurs ont identifié trois régions de la survivine contenant des épitopes T CD4+ restreints aux 12 molécules HLA II prépondérantes dans la population caucasienne. Ces peptides représentent des candidats potentiels pour la vaccination prophylactique ou thérapeutique contre les cancers, étant donné qu'ils sont susceptibles d'induire une réponse T CD4+ dirigée contre la tumeur, chez la majorité des patients vaccinés, dans la mesure où : (i) ils sont issus d'un antigène exprimé par la majorité des cellules tumorales, (ii) ils sont aptes à induire des lymphocytes T CD4+ spécifiques de cet antigène, et (iii) ils prennent en compte le polymorphisme des molécules HLA II.

En outre, ces peptides qui sont reconnus par des lymphocytes T CD4+ spécifiques d'un antigène tumoral exprimé par la majorité des cellules tumorales, sont utiles pour l'établissement du pronostic et le suivi de l'évolution des cancers.

La présente invention a en conséquence pour objet un peptide dérivé de l'isoforme alpha de la survivine pour utilisation comme antigène dans la vaccination prophylactique ou thérapeutique, le diagnostic, le pronostic ou le suivi thérapeutique du cancer tel que défini par les revendications. Lequel peptide étant sélectionné dans le groupe constitué par :
a) les peptides de 13 à 18 acides aminés consécutifs situés entre les positions 17 et 34 de l'isoforme alpha de la survivine,
b) les peptides de 13 à 30 acides aminés consécutifs situés entre les positions 84 et 113 de l'isoforme alpha de la survivine, et
c) les peptides de 13 à 21 acides aminés consécutifs situés entre les positions 122 et 142 de l'isoforme alpha de la survivine,
lesquels peptides en a), b) et c), présentant une activité de liaison à au moins quatre molécules HLA-II prépondérantes dans la population caucasienne, inférieure à 1000 nM, et étant capables d'induire des lymphocytes T CD4+ spécifiques de la survivine.

### Définitions

On entend par « peptide apte à être présenté par au moins une molécule HLA II » un peptide possédant une activité de liaison à au moins une molécule HLA-II, inférieure à 1000 nM.

On entend par « survivine », les trois isoformes de la survivine : isoforme alpha, survivine-2B et survivine ΔEx-3. Ces isoformes peuvent être issues de n'importe quel mammifère; de préférence, il s'agit des isoformes humaines. L'isoforme alpha de la survivine correspond à la survivine de 142 acides aminés ; les positions sont indiquées en référence à la séquence humaine (Genbank AAC51660 ou SwissProt O15392).

On entend par « molécule HLA II prépondérante dans la population caucasienne » ou « molécule HLA II prépondérante », une molécule HLA II comprenant une chaîne béta codée par un allèle dont la fréquence est supérieure à 5 % dans la population caucasienne, comme précisé au Tableau 1 ci-dessus. Certaines des molécules HLA II prépondérantes dans la population caucasienne et notamment les molécules HLA-DP401 et HLA-DP402 sont également prépondérantes dans d'autres populations (Amérique du Sud, Inde, Japon, Afrique ; Tableau I). En conséquence, les peptides selon l'invention ne sont pas restreints à une utilisation dans la population caucasienne, et ils peuvent également être utilisés pour vacciner des individus de pays autres que ceux d'Amérique du Nord et d'Europe, dans lesquels lesdites molécules HLA II sont prépondérantes, comme précisé au Tableau I.

On entend par « cancer », un cancer associé à la surexpression de la survivine tel que de façon non-limitative : les cancers du sein, foie, colon, poumon, ovaire, utérus, oesophage, estomac, pancréas, foie et prostate, les mélanomes, la maladie de Hodgkin, les lymphomes non hodgkiniens, les leucémies, le syndrome myélodisplasique avec anémie réfractaire, les neuroblastomes, les phéochromocytomes, les sarcomes des tissus mous et les tumeurs cérébrales.

Les peptides selon l'invention comprennent un épitope T CD4+ de la survivine apte à être présenté à au moins une molécule HLA II prépondérante, lequel épitope est constitué par une séquence de 9 acides aminés incluant les résidus d'ancrage aux molécules HLA II, flanquée à l'une de ses extrémités, de préférence aux deux extrémités, par au moins 2 acides aminés, de préférence 3 acides aminés.

Les peptides selon l'invention présentent les propriétés suivantes :
- activité de liaison aux molécules HLA II: les peptides selon l'invention possèdent une bonne affinité pour les molécules HLA II prépondérantes, notamment dans la population caucasienne (activité de liaison < 1000 nM) ; l'activité de liaison est définie par le test décrit dans la revendication 1. L'activité peut-être mesurée par un test de liaison HLA II/peptide, en compétition, avec une révélation immuno-enzymatique, selon le principe décrit dans le brevet américain US 6,649,166 et la Demande Internationale PCT WO 03/040299, respectivement pour les molécules HLA-DR et HLA-DP4.
- immunogénicité : Ces peptides sont également capables d'induire des cellules T CD4+ spécifiques de la survivine à partir des précurseurs présents chez la majorité des individus naïfs ou bien de stimuler de telles cellules chez la majorité des individus atteints d'un cancer associé à la surexpression de la survivine. L'immunogénicité des peptides peut être déterminée, notamment à partir de cellules mononucléées du sang périphérique (PBMCs), par tout essai approprié connu de l'Homme du métier comme par exemple : un test de prolifération cellulaire, un test ELISPOT (dosage des cellules productrices de cytokines) ou un test de dosage des cytokines intracellulaires (IFN-γ, IL-2, IL-4 et IL-10).

La description dévoile des variants naturels ou synthétiques obtenus par mutation (insertion, délétion, substitution) d'un ou plusieurs acides aminés dans la séquence de l'isoforme alpha de la survivine humaine, dès lors que ledit variant conserve une bonne affinité pour au moins une molécule HLA II prépondérante, notamment dans la population caucasienne (activité de liaison < 1000 nM) et est immunogène. Les variants naturels résultent notamment du polymorphisme de la survivine. En outre, d'autres variants peuvent être aisément construits étant donné que les résidus d'acides aminés impliqués dans la liaison aux molécules HLA- DR et HLA-DP4 (résidus d'ancrage) et l'effet des modifications de ces résidus sur la liaison aux molécules HLA-DR et HLA-DP4 sont connus de l'Homme du métier ; la Demande Internationale PCT WO 03/040299 enseigne notamment que pour lier HLA-DP4, le résidu en P6 doit être aromatique ou hydrophobe ou consister en un résidu de cystéine (C), et au moins l'un des résidus P1, P9 est tel que P1 est aromatique ou hydrophobe et/ou P9 est aromatique ou hydrophobe ou consiste en un résidu C, D, Q, S, T ou E, alors que le résidu en P4 peut être n'importe quel résidu d'acide aminé. Le Brevet américain US 6,649,166 décrit une méthode générale permettant de déterminer les résidus d'ancrage aux molécules HLA DR (P1, P4, P6, P7 et P9) et la nature des mutations de ces résidus permettant de modifier l'affinité pour les molécules HLA DR. Des motifs de liaison aux molécules HLA DR sont décrits notamment dans Sturnolio et al., Nat. Biotech, 1999, 17, 533-534 et Rammensee et al., Immunogenetics, 1995, 41, 178-228.

La description englobe également les peptides modifiés dérivés des précédents par introduction de toute modification au niveau de résidu(s) d'acide aminé, de la liaison peptidique ou des extrémités des peptides, dès lors que ledit peptide modifié conserve une bonne affinité pour au moins une molécule HLA II prépondérante, notamment dans la population caucasienne (activité de liaison < 1000 nM) et est immunogène. Ces modifications qui sont introduites dans les peptides par les méthodes classiques connues de l'Homme du métier, incluent de façon non-limitative : la substitution d'un acide aminé par un acide aminé non-protéinogénique (acide aminé D ou analogue d'acide aminé); l'addition de groupement chimique (lipide, oligo ou polysaccharide) au niveau d'une fonction réactive, notamment de la chaîne latérale R ; la modification de la liaison peptidique (-CO-NH-), notamment par une liaison du type rétro ou rétro-inverso (-NH-CO-) ou une liaison différente de la liaison peptidique ; la cyclisation; la fusion d'un peptide (épitope d'intérêt pour la vaccination ; étiquette utile pour la purification du peptide, notamment sous forme clivable par une protéase); la fusion de la séquence dudit peptide avec celle d'une protéine, notamment une chaîne α ou β d'une molécule HLA II ou le domaine extra-cellulaire de ladite chaîne ou bien encore une séquence de ciblage dans l'endosome dérivée notamment de la chaîne invariable Ii ou de la protéine LAMP-1 ; le couplage à une molécule appropriée, notamment un marqueur, par exemple un fluorochrome. Ces modifications sont destinées en particulier à augmenter la stabilité et plus particulièrement la résistance à la protéolyse, ainsi que la solubilité, l'immunogénicité ou à faciliter la purification ou la détection, soit du peptide selon l'invention, soit de cellules CD4+ spécifiques dudit peptide.

Ladite molécule HLA II prépondérante dans la population caucasienne est choisie parmi les molécules HLA-DR1, HLA-DR3, HLA-DR4, HLA-DR7, HLA-DR11, HLA-DR13, HLA-DR15, HLA-DRB3, HLA-DRB4, HLA-DRB5 et HLA-DP4, de préférence parmi les molécules HLA-DR1, HLA-DR4, HLA-DR7, HLA-DR11, HLA-DR13, HLA-DR15, HLA-DRB5, HLA-DP401 et HLA-DP402.

Lesdites molécules HLA II sont codées respectivement par les allèles HLA DRB1*0101, DRB1*0301, DRB1*0401, DR1*0701, DRB1*1101, DRB1*1301, DRB1*1501, DRB3*0101, DRB4*0101, DRB5*0101, DP*0401 et DP*0402.

Parmi ces molécules HLA II prépondérantes dans la population caucasienne, les molécules HLA-DP401 et HLA-DP402 sont également prépondérantes en Amérique du Sud, en Inde, en Afrique et au Japon.

De manière particulièrement avantageuse, ledit peptide est apte à être présenté par au moins une molécule HLA-DP401 ou HLA-DP402.

Le peptide est sélectionné dans le groupe constitué par les peptides : 17-31, 19-33, 20-34, 84-98, 90-104, 91-105, 93-107, 96-110 et 128-142 qui possèdent une bonne affinité (activité de liaison < 1000 nM) pour au moins quatre molécules HLA II prépondérantes dans la population caucasienne.

De manière remarquable, l'ensemble des peptides qui possèdent une bonne affinité pour au moins quatre molécules HLA II prépondérantes dans la population caucasienne, à l'exception du peptide 128-142, possèdent également une bonne affinité pour au moins une molécule HLA-DP401 ou HLA-DP-402.

Ledit peptide est avantageusement sélectionné dans le groupe constitué par les séquences SEQ ID NO : 5, 6, 7, 17, 19, 20, 21, 23 et 28.

De tels peptides permettent à la fois d'obtenir une bonne couverture vaccinale chez les individus de la population caucasienne et d'élargir la couverture vaccinale aux individus d'autres populations (Amérique du Sud, Afrique, Inde et Japon).

De manière préférée, ledit peptide est sélectionné dans le groupe constitué par les peptides : 17-31, 19-33, 90-104, 93-107, 96-110 et 128-142.

De manière encore plus préférée, ledit peptide est sélectionné dans le groupe constitué par les peptides 19-33, 90-104 et 93-107.

Selon un autre mode de réalisation avantageux dudit peptide, il présente une séquence de 13, 14, 15, 16, 17, 18, 19, 20, 21, 22, 23, 24 ou 25 acides aminés.

La description décrit un variant d'un des peptides précédents, obtenu par la substitution d'au moins un des résidus P1, P6 et/ou P9 d'ancrage aux molécules HLA II, par un acide aminé aromatique ou hydrophobe, du résidu P6 par une cystéine (C), du résidu P9 par C, D, Q, S, T ou E et/ou du résidu P4 par un autre acide aminé naturel ou synthétique.

On entend par acide aminé naturel ou synthétique, les 20 α-acides aminés naturels communément trouvés dans les protéines (A, R, N, D, C, Q, E, G, H, I, L, K, M, F, P, S, T, W, Y et V), certains acides aminés rarement rencontrés dans les protéines (hydroxyproline, hydroxylysine, méthyllysine, diméthyllysine..), les acides aminés qui n'existent pas dans les protéines tels que la β-alanine, l'acide γ-amino-butyrique, l'homocystéine, l'ornithine, la citrulline, la canavanine, la norleucine, la cyclohexylalanine..., ainsi que les énantiomères et les diastéréoisomères des acides aminés précédents.

On entend par acide aminé hydrophobe, un acide aminé sélectionné parmi (code à une lettre) : A, V, L, I, P, W, F et M.

On entend par acide aminé aromatique, un acide aminé sélectionné parmi (code à une lettre) : F, W et Y.

Selon un autre mode de réalisation avantageux dudit peptide, il est marqué ou complexé ; il peut être notamment complexé à des molécules HLA II marquées, par exemple biotinylées, pour former des complexes HLA II/peptides, en particulier des complexes multimériques comme des tétramères.

La présente invention a également pour objet un fragment polyépitopique comprenant la concaténation d'au moins deux épitopes identiques ou différents dont l'un au moins est un épitope T CD4+ de la survivine inclus dans un peptide de l'invention, pour utilisation comme antigène dans la vaccination prophylactique ou thérapeutique, le diagnostic, le pronostic ou le suivi thérapeutique du cancer.

De préférence, ledit fragment polyépitopique présente une longueur de 20 à 1000 acides aminés, de préférence de 20 à 100 acides aminés.

Ledit fragment polyépitopique comprend avantageusement une étiquette fusionnée à l'une de ses extrémités, pour la purification ou la détection dudit fragment. L'étiquette, notamment une séquence polyhistidine ou un épitope B d'un antigène, est de préférence séparée de la séquence polyépitopique par un site de clivage d'une protéase de façon à isoler la séquence polyépitopique, à partir de la fusion.

Selon un mode de réalisation avantageux dudit fragment polyépitopique, il comprend la concaténation d'au moins un épitope T CD4+ de la survivine inclus dans un peptide de l'invention et d'au moins un épitope sélectionné dans le groupe constitué par :
- un épitope T CD8+ de la survivine, présenté par une molécules HLA I et reconnu par des lymphocytes T cytotoxiques spécifiques dudit antigène ; de tels épitopes T CD8+ sont notamment décrits sur le site http://www.cancerimmunity.org/peptidedatabase/tumorspecific.htm; de préférence l'épitope T CD8+ est choisi parmi : survivine 96-104 (LTLGEFLKL, SEQ ID NO : 37) ou 95-104 (ELTLGEFLKL, SEQ ID NO: 38), survivine-2B 80-88 (AYACNTSTL, SEQ ID NO : 39) et les peptides tels que décrits dans le Tableau I de Bachinsky et al., Cancer Immun., 2005, 5, 6.
- un épitope T CD4+ universel naturel ou synthétique tel que le peptide de la toxine tétanique TT 830-846 (O'SULLIVAN et al., J. Immunol., 1991, 147, 2663-2669), le peptide de l'hémagglutinine du virus de la grippe HA 307-319 (O'Sullivan et al, précité), le peptide PADRE (KXVAAWTLKAA, SEQ ID NO : 41; Alexander et al., Immunity, 1994, 1, 751-761) et des peptides issus des antigènes de *Plasmodium falciparum* tels que le peptide CS.T3 (Sinigaglia et al., Nature, 1988, 336, 778-780) et les peptides CSP, SSP2, LSA-1 et EXP-1 (Doolan et al., J. Immunol., 2000, 165, 1123-1137),
- un épitope B constitué par un sucre (Alexander et al., précité), ledit épitope B étant de préférence sous la forme d'un glycopeptide, et
- un épitope B de la survivine reconnu spécifiquement par des anticorps dirigés contre ledit antigène tumoral.

La combinaison de , l'épitope T CD4+ avec l'un au moins des épitopes de l'invention permet avantageusement de déclencher ou de moduler une réponse immunitaire anti-tumorale.

La présente invention a également pour objet un lipopeptide comprenant un peptide ou un fragment polyépitopique de l'invention, pour utilisation comme antigène dans la vaccination prophylactique ou thérapeutique, le diagnostic, le pronostic ou le suivi thérapeutique du cancer.

Ledit lipopeptide est obtenu par addition d'un lipide sur une fonction α-aminée ou sur une fonction réactive de la chaîne latérale d'un acide aminé dudit peptide ou fragment polyépitopique de l'invention; il peut comprendre une ou plusieurs chaînes dérivées d'acides gras en C₄₋₂₀, éventuellement ramifiées ou insaturées (acide palmitique, acide oléique, acide linoléique, acide linolénique, acide 2-amino hexadécanoïque, pimélautide, trimétauxide) ou un dérivé d'un stéroïde. La partie lipidique préférée est notamment représentée par un groupe N^{α}-acétyl-lysine N^{ε}(palmitoyl), également dénommé Ac-K(Pam).

La présente invention a également pour objet une protéine de fusion constituée par une protéine ou un fragment de protéine, fusionnés avec un peptide ou un fragment polyépitopique de l'invention, pour utilisation comme antigène dans la vaccination prophylactique ou thérapeutique, le diagnostic, le pronostic ou le suivi thérapeutique du cancer.

Le peptide ou le fragment polyépitopique peuvent être fusionnés avec l'extrémité NH₂ ou COOH de ladite protéine ou insérés dans la séquence de ladite protéine.

Selon un mode de réalisation avantageux de ladite protéine de fusion, elle est constituée par un peptide de l'invention, fusionné avec une séquence de ciblage dans l'endosome, dérivée de préférence d'une chaîne invariable humaine Ii ou de la protéine LAMP-1. Les séquences de ciblage dans l'endosome et leur utilisation pour le ciblage d'antigènes dans l'endosome sont notamment décrites dans Sanderson et al. (P.N.A.S., 1995, 92, 7217-7222), Wu et al. (P.N.A.S., 1995, 92, 11671-11675) et Thompson et al. (J. Virol., 1998, 72, 2246-2252).

Selon un autre mode de réalisation avantageux de ladite protéine de fusion, elle est constituée par un peptide tel que défini dans la revendication 1, fusionne avec l'une des chaînes d'une molécule HLA II, de préférence la chaîne béta, ou bien avec un fragment de celle-ci correspondant à une molécule HLA II soluble, notamment un fragment correspondant au domaine extracellulaire précédé du peptide signal homologue ou d'un peptide signal hétérologue. Ledit peptide est avantageusement inséré entre le peptide signal et l'extrémité NH₂ du domaine extracellulaire de la chaîne β, comme décrit pour la molécule HLA-DR (Kolzin et al., PNAS, 2000, 97, 291-296).

Alternativement, le peptide ou le fragment polyépitopique de l'invention sont fusionnés avec une protéine facilitant leur purification ou leur détection, connue de l'Homme du métier comme notamment la Glutathione-S- Transférase (GST) et les protéines fluorescentes (GFP et dérivées). Dans ce cas, la séquence du peptide ou du fragment polyépitopique d'intérêt est de préférence séparée du reste de la protéine par un site de clivage d'une protéase, pour faciliter la purification dudit peptide ou dudit fragment polyépitopique.

La présente invention a également pour objet un vecteur d'expression comprenant un polynucléotide codant pour un peptide, un fragment polyépitopique ou une protéine de fusion de l'invention, sous le contrôle de séquences régulatrices appropriées, de la transcription et éventuellement de la traduction, pour utilisation comme antigène dans la vaccination prophylactique ou thérapeutique, le diagnostic ou le pronostic du cancer.

Conformément à l'invention, la séquence dudit polynucléotide est celle de l'ADNc codant pour ledit peptide ou fragment polyépitopique ou ladite protéine de fusion. Ladite séquence peut avantageusement être modifiée de façon à ce que l'usage des codons soit optimal chez l'hôte dans lequel elle est exprimée. En outre, ledit polynucléotide peut être lié à au moins une séquence hétérologue.

Au sens de la présente invention, on entend par séquence hétérologue relativement à une séquence d'acide nucléique codant pour la survivine, toute séquence d'acide nucléique autre que celles qui, dans la nature, sont immédiatement adjacentes à ladite séquence d'acide nucléique codant ledit peptide de la survivine.

Conformément à l'invention ledit vecteur recombinant comprend une cassette d'expression incluant au moins un polynucléotide tel que défini ci-dessus, sous le contrôle de séquences régulatrices de la transcription et éventuellement de la traduction appropriées (promoteur, activateur, intron, codon d'initiation (ATG), codon stop, signal de polyadénylation),

De nombreux vecteurs dans lesquels on peut insérer une molécule d'acide nucléique d'intérêt afin de l'introduire et de la maintenir dans une cellule hôte eucaryote ou procaryote, sont connus en eux-mêmes ; le choix d'un vecteur approprié dépend de l'utilisation envisagée pour ce vecteur (par exemple réplication de la séquence d'intérêt, expression de cette séquence, maintien de cette séquence sous forme extrachromosomique, ou bien intégration dans le matériel chromosomique de l'hôte), ainsi que de la nature de la cellule hôte. Par exemple, on peut utiliser entre autres des vecteurs viraux tels que les adénovirus, les rétrovirus, les lentivirus, les AAV et les baculovirus, dans lesquels a été insérée préalablement la séquence d'intérêt ; on peut également associer ladite séquence (isolée ou insérée dans un vecteur plasmidique) avec une substance lui permettant de franchir la membrane des cellules hôtes, telle qu'un transporteur comme un nanotransporteur ou une préparation de liposomes, ou de polymères cationiques, ou bien l'introduire dans ladite cellule hôte en utilisant des méthodes physiques telles que l'électroporation ou la microinjection. En outre, on peut avantageusement combiner ces méthodes, par exemple en utilisant l'électroporation associée à des liposomes.

La présente invention a également pour objet une composition immunogène ou vaccinale, caractérisée en ce qu'elle comprend au moins un peptide, un fragment polyépitopique, une protéine de fusion, un lipopeptide ou un vecteur tels que définis par l'invention, et un vehicule pharmaceutiquement acceptable, une substance porteuse ou un adjuvant.

La composition immunogène selon l'invention se présente sous une forme galénique adaptée à une administration par voie parentérale (sous-cutanée, intramusculaire, intraveineuse), entérale (orale, sublinguale), ou locale (rectale, vaginale).

Les véhicules pharmaceutiquement acceptables, les substances porteuses et les adjuvants sont ceux classiquement utilisés.

Les adjuvants sont avantageusement choisis dans le groupe constitué par : des émulsions huileuses, des substances minérales, des extraits bactériens, la saponine, l'hydroxyde d'alumine, le monophosphoryl -lipide A et le squalène.

Les substances porteuses sont avantageusement sélectionnées dans le groupe constitué par : les liposomes unilamellaires ou multilamellaires, les ISCOMS, les virosomes, les pseudoparticules virales, les micelles de saponine, les microsphères solides de nature saccharidique (poly(lactide-co-glycolide)) ou aurifère, et les nanoparticules.

Selon un mode de réalisation avantageux de ladite composition, elle comprend au moins un épitope T CD4+ et un épitope T CD8+ de la survivine, sous forme d'un mélange de peptides, d'un fragment polyépitopique et/ou d'un vecteur d'expression codant lesdits peptides ou ledit fragment, tels que définis par les revendications.

Selon une disposition avantageuse de ce mode de réalisation de ladite composition, l'épitope T CD8+ est choisi parmi : survivine 96-104 (LTLGEFLKL) ou 95-104 (ELTLGEFLKL), survivine-2B 80-88 (AYACNTSTL) et les peptides tels que décrits dans le Tableau I de Bachinsky et al., Cancer Immun., 2005, 5, 6.

Selon un autre mode de réalisation avantageux de ladite composition, elle comprend au moins deux peptides de la survivine incluant un épitope T CD4+ tels que défini ci-dessus, sélectionnés dans le groupe constitué par l'une des combinaisons suivantes:
- le peptide 17-31 et l'un au moins des peptides peptide 19-33, 90-104 ou 128-142
- le peptide 19-33 et le peptide 96-110,
- le peptide 90-104 et le peptide 17-31,
- le peptide 96-110 et le peptide 90-104, et
- les peptides 93-107 et 128-142, et l'un au moins des peptides 17-31, 19-33, 96-110 ou 90-104.

De telles combinaisons permettent avantageusement d'induire des lymphocytes T CD4+ chez la quasi-totalité des individus vaccinés.

Selon encore un autre mode de réalisation avantageux de ladite composition, elle comprend un peptide incluant un épitope T CD4+ universel, tel que défini ci-dessus.

La présente invention a également pour objet l'utilisation d'au moins un peptide, un fragment polyépitopique, une protéine de fusion, un lipopeptide et/ou un vecteur tels que définis par les revendications pour la préparation d'un vaccin destiné à la prévention ou au traitement du cancer.

Les peptides selon la présente invention et les produits dérivés (fragment polyépitopique, protéine de fusion, lipopeptide, vecteur recombinant) peuvent être utilisés en immunothérapie dans le traitement des tumeurs surexprimant la survivine. Lesdits peptides ou produits dérivés sont utilisés, soit comme vaccin, soit en thérapie cellulaire, ou bien encore par une combinaison des deux approches.

La thérapie cellulaire comprend, la préparation de cellules présentatrices d'antigènes (cellules dendritiques) par un protocole classique comprenant l'isolement de cellules mononucléées du sang périphérique (PBMC) d'un patient à traiter et la mise en culture des cellules dendritiques en présence de peptide(s). Dans une seconde étape les cellules présentatrices d'antigène chargées avec le peptide sont réinjectées au patient.

La présente invention a également pour objet l'utilisation d'au moins un peptide, un fragment polyépitopique, une protéine de fusion, un lipopeptide et/ou un vecteur de l'invention pour la préparation d'un réactif de diagnostic, d'évaluation du pronostic ou de suivi du traitement d'un cancer. Ledit réactif comprend un peptide ou une protéine de fusion tels que définis par les revendications, éventuellement marqués ou complexés, notamment complexés à des molécules HLA II marquées, par exemple biotinylées, sous la forme de complexes multimériques comme des tétramères.

La présente invention a également pour objet une méthode *in vitro,* de diagnostic, d'évaluation du pronostic ou de suivi du traitement d'un cancer chez un individu, caractérisée en ce qu'elle comprend :
- la mise en contact d'un échantillon biologique dudit individu avec un peptide de l'invention, et
- la détection de lymphocytes T CD4+ spécifiques de la survivine, par tout moyen approprié.

La présente invention a également pour objet un kit de diagnostic, d'évaluation du pronostic ou de suivi du traitement d'un cancer, caractérisé en ce qu'il comprend au moins un peptide tel que défini par l'invention et éventuellement un réactif de détection de lymphocytes T CD4+.

La méthode selon l'invention permet de suivre l'évolution de la réponse T CD4+ dirigée contre la survivine au cours d'un cancer ou bien d'un traitement antitumoral, notamment une immunothérapie antitumorale ; les lymphocytes T CD4+ spécifiques de la survivine peuvent être de type TH1 (sécrétion d'IFN-γ), TH2 (sécrétion d'IL-4) ou T régulateur (sécrétion d'IL-10 ou de TGF-β); il est attendu que la réponse T de type TH1 est signe d'une évolution favorable du cancer alors que la réponse T régulatrice est signe d'une évolution défavorable de ce cancer. La détection est effectuée à partir d'un échantillon biologique contenant des cellules T CD4+, notamment un échantillon de cellules mononucléées isolées à partir d'un prélèvement de sang périphérique (PBMCs).

Les lymphocytes T CD4+ spécifiques de la survivine sont détectés par tous moyens, connus en eux-mêmes. Par exemple, on peut utiliser des moyens directs comme la cytométrie de flux en présence de complexes multimériques tels que définis ci-dessus, ou bien des moyens indirects comme les tests de prolifération lymphocytaire et les dosages de cytokines telles que l'IL-2, l'IL-4, l'IL-5, IL-10 et l'IFN-γ, notamment par des techniques immunoenzymatiques (ELISA, RIA, ELISPOT) ou par cytométrie de flux (dosage des cytokines intracellulaires).

De manière plus précisé :
Une suspension de cellules (PBMC, PBMC déplétées en cellules CD8+, lymphocytes T préalablement enrichis par une étape de culture *in vitro* avec les peptides tels que définis ci-dessus ou des lymphocytes T clonés) est cultivée pendant 3 à 5 jours en présence desdits peptides et au besoin de cellules présentatrices appropriées, telles que des cellules dendritiques, des PBMC autologues ou hétérologues, des cellules lymphoblastoïdes telles que celles obtenues après infection par le virus EBV ou des cellules génétiquement modifiées. La présence de cellules T CD4+ spécifiques de la survivine, dans la suspension initiale est détectée à l'aide des peptides, selon l'une des méthodes suivantes :

### * test de prolifération :

La prolifération des cellules T CD4+ spécifiques de la survivine est mesurée par incorporation de thymidine tritiée dans l'ADN des cellules.

### * test ELISPOT :

Le test ELISPOT permet de révéler la présence de cellules T sécrétant de cytokines (IL-2, l'IL-4, l'IL-5, IL-10 et l'IFN-γ), spécifiques d'un peptide tel que défini ci-dessus. Le principe de ce test est décrit dans Czerkinsky et al., J. Immunol. Methods, 1983, 65, 109-121 et Schmittel et al., J. Immunol. Methods, 1997, 210, 167-174, et sa mise en oeuvre est illustrée dans la Demande Internationale WO 99/51630 ou Gahéry-Ségard et al., J. Virol., 2000, 74, 1694-1703.

### * détection des cytokines :

La présence de cellules T spécifiques de la survivine, sécrétant des cytokines telles que l'IL-2, l'IL-4, l'IL-5, IL-10 et l'IFN-γ est détectée, soit par dosage des cytokines présentes dans le surnageant de culture, par un test immunoenzymatique, notamment à l'aide d'un kit commercial, soit par détection des cytokines intracellulaires en cytométrie de flux. Le principe de détection des cytokines intracellulaires est décrit dans Goulder et al. , J. Exp. Med., 2000, 192, 1819-1832 et Maecker et al., J. Immunol. Methods, 2001, 255, 27-40 et sa mise en oeuvre est illustrée dans Draenert et al., J. Immunol. Methods, 2003, 275, 19-29.

### * complexes multimériques

- un échantillon biologique, de préférence des cellules mononucléées du sang périphérique (PBMC), est mis en contact avec des complexes multimériques marqués, notamment par un fluorochrome, formés par la liaison entre des molécules HLA II solubles et des peptides tels que définis ci-dessus, et
- les cellules marquées par lesdits complexes multimériques sont analysées, notamment par cytométrie de flux.

De manière avantageuse, préalablement à la mise en contact de l'échantillon biologique avec lesdits complexes, on l'enrichit en cellules T CD4+, en le mettant en contact avec des anticorps anti-CD4.

Les complexes multimériques HLA II/peptide peuvent être préparés à partir de molécules natives extraites de cellules exprimant une molécule HLA II ou de molécules recombinantes produites dans des cellules hôte appropriées comme précisé, par exemple dans NOVAK et al. (J. Clin. Investig., 1999, 104, R63-R67) ou dans KURODA et al. (J. Virol., 2000, 74, 18, 8751-8756). Ces molécules HLA II peuvent notamment être tronquées (délétion du domaine transmembranaire) et leur séquence peut-être modifiée afin de les rendre solubles ou bien de faciliter l'appartement des chaînes alpha et béta (Novak et al., précité).

Le chargement de molécules HLA II avec le peptide peut se faire par mise en contact d'une préparation de molécules HLA II comme ci-dessus, avec le peptide. Par exemple, des molécules HLA II solubles et biotinylées sont incubées, pendant 72 heures à 37°C, avec un excès d'un facteur 10 de peptides tels que définis ci-dessus, dans un tampon phosphate-citrate 10 mM, NaCl 0,15 M à un pH compris entre 4,5 et 7.

Alternativement, la séquence du peptide peut être introduite dans l'une des chaînes de la molécule HLA II sous forme d'une protéine de fusion qui permet la préparation de complexes multimériques HLA II/peptides à partir de cellules hôtes appropriées exprimant ladite protéine de fusion. Lesdits complexes peuvent ensuite être marqués, notamment par la biotine.

Les complexes multimériques de type tétramère sont notamment obtenus en ajoutant aux molécules HLA II chargées, de la streptavidine marquée par un fluorochrome en quantité quatre fois moindre (mole à mole) par rapport aux molécules HLA II. L'ensemble étant ensuite incubé pendant une durée suffisante, par exemple une nuit à température ambiante.

Les complexes multimériques peuvent également être formés, soit par incubation de monomères HLA II/peptides avec des billes magnétiques couplées à la streptavidine comme décrit pour les molécules HLA I (Bodinier et al., Nature, 2000, 6, 707-710), soit par insertion de monomères HLA II/peptides dans des vésicules lipidiques comme décrit pour les molécules du CMH de classe II murines (Prakken, Nature Medicine, 2000,6, 1406-1410).

Pour utiliser ces complexes multimériques HLA II/peptide notamment de type tétramère, on met en contact une suspension de cellules (PBMC, PBMC déplétées en cellules CD8+, lymphocytes T préalablement enrichis par une étape de culture *in vitro* avec des peptides tels que définis ci-dessus ou des lymphocytes T clonés) avec des complexes multimériques HLA II/peptide à une concentration appropriée (par exemple de l'ordre de 10 à 20 µg/ml), pendant une durée suffisante pour permettre la liaison entre les complexes et les lymphocytes T CD4+ spécifiques de la survivine (par exemple, de l'ordre de 1 à 3 heures). Après lavage, la suspension est analysée par cytométrie de flux : on visualise le marquage des cellules par les complexes multimériques qui sont fluorescents.

La cytométrie de flux permet de séparer les cellules marquées par les complexes multimériques HLA II/peptide des cellules non marquées et d'effectuer ainsi un tri cellulaire.

La présente invention a ainsi, en outre, pour objet un procédé de tri de lymphocytes T CD4+ spécifiques de la survivine, caractérisé en ce qu'il comprend au moins les étapes suivantes :
- la mise en contact, *in vitro,* d'un échantillon cellulaire avec des complexes multimériques HLA II/peptide marqués, notamment par un fluorochrome, lesdits complexes étant formés par la liaison de molécules HLA II solubles avec au moins un peptide de l'invention, et
- le tri des cellules liées auxdits complexes HLA II/peptide, notamment en cytométrie de flux.

La présente invention a en outre pour objet un peptide tel que défini dans les revendications, à l'exclusion du peptide situé entre les position 89 à 101 de l'isoforme alpha de la survivine (peptide 89-101).

La présente invention a également pour objet un fragment polyépitopique, une protéine de fusion, un lipopeptide, un polynucléotide, un vecteur recombinant et une cellule hôte procaryote ou eucaryote modifiée, tels que définis dans les revendications.

Les polynucléotides, les vecteurs recombinants et les cellules transformées tels que définis ci-dessus, sont utiles notamment pour la production des peptides, fragments polyépitopiques et protéines de fusion selon l'invention.

Les polynucléotides selon l'invention sont obtenus par les méthodes classiques, connues en elles-mêmes, en suivant les protocoles standards tels que ceux décrits dans Current Protocols in Molecular Biology (Frederick M. A USUBEL, 2000, Wiley and son Inc, Library of Congress, USA*).* Par exemple, ils peuvent être obtenus par amplification d'une séquence nucléique par PCR ou RT-PCR, par criblage de banques d'ADN génomique par hybridation avec une sonde homologue, ou bien par synthèse chimique totale ou partielle. Les vecteurs recombinants sont construits et introduits dans des cellules hôtes par les méthodes classiques d'ADN recombinant et de génie génétique, qui sont connues en elles-mêmes.

Les peptides et leurs dérivés (variants, peptides modifiés, lipopeptides, fragments polyépitopiques, protéines de fusion), sont préparés par les techniques classiques connues de l'Homme du métier, notamment par synthèse en phase solide ou liquide ou par expression d'un ADN recombinant dans un système cellulaire approprié (eucaryote ou procaryote).

De manière plus précise :
- les peptides et leurs dérivés (variants, fragments polyépitopiques) peuvent être synthétisés en phase solide, selon la technique Fmoc, originellement décrite par Merrifield et al. (J. Am. Chem. Soc., 1964, 85: 2149-) et purifiés par chromatographie liquide haute performance en phase inverse,
- les lipopeptides peuvent être notamment préparés selon le procédé décrit dans les Demandes Internationales WO 99/40113 ou WO 99/51630.
- les peptides et dérivés tels que les variants, les fragments polyépitopiques et les protéines de fusion peuvent également être produits à partir des ADNc correspondants, obtenus par tout moyen connu de l'homme du métier ; l'ADNc est cloné dans un vecteur d'expression eucaryote ou procaryote et la protéine ou le fragment produits dans les cellules modifiées par le vecteur recombinant sont purifiés par tout moyen approprié, notamment par chromatographie d'affinité.

Outre les dispositions qui précèdent, l'invention comprend encore d'autres dispositions, qui ressortiront de la description qui va suivre, qui se réfère à des exemples de mise en oeuvre de l'objet de la présente invention, avec référence au dessin annexé dans lequel:

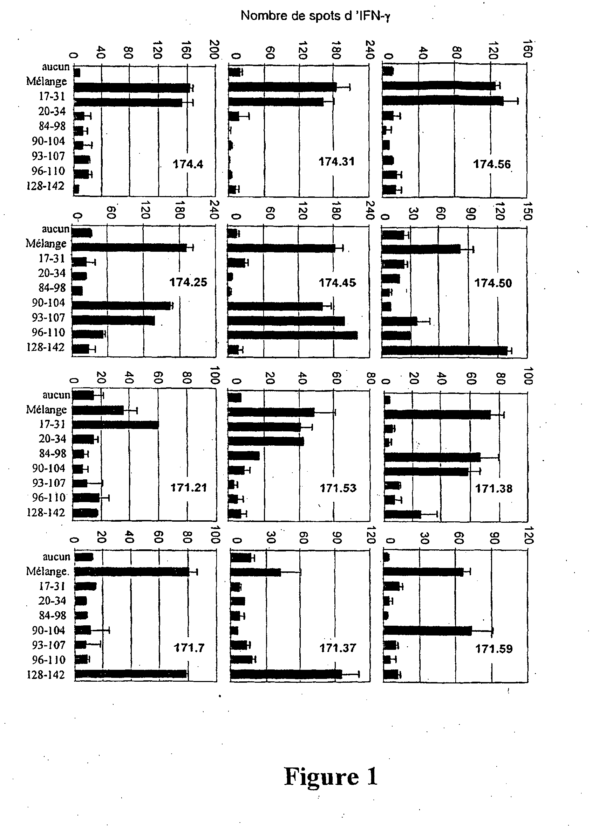

- la figure 1 illustre la spécificité des lignées de lymphocytes T CD4+ obtenues à partir des PBMC des donneurs sains 171 et 174. Les lignées de lymphocytes T CD4+ ont été obtenues après trois stimulations à une semaine d'intervalle, avec des cellules dendritiques autologues chargées avec un mélange des sept peptides sélectionnés (17-31, 20-34, 84-98, 90-104, 93-107, 96-110 et 128-142). La spécificité des lignées de lymphocytes T a été évaluée par ELISPOT IFN-γ. 10⁴ lymphocytes T CD4+ ont été incubés, en duplicat, avec 10⁵ PBMC autologues, en présence ou en l'absence de peptide. La révélation des spots a été effectuée 24 h après l'incubation. Chaque barre représente le nombre moyen de spots des duplicats ± écart type. Les valeurs positives sont au moins trois fois supérieures à celles du contrôle négatif.

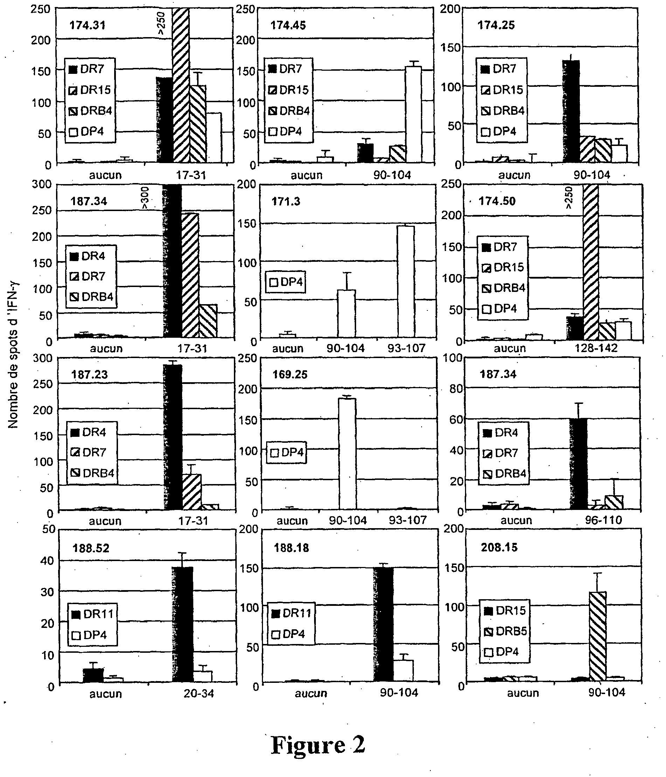

- la figure 2 illustre les éléments de restriction HLA II des lignées de lymphocytes T CD4+ spécifiques de différents peptides de la survivine, issues de six donneurs sains (169, 171, 174, 187, 188, 208). Les lignées de lymphocytes T CD4+ on été obtenues après trois stimulations à une semaine d'intervalle, avec des cellules dendritiques autologues chargées avec un mélange des sept peptides sélectionnés (17-31, 20-34, 84-98, 90-104, 93-107, 96-110 et 128-142). La restriction HLA II des lignées de lymphocytes T a été évaluée par ELISPOT IFN-γ. 10⁴ lymphocytes T CD4+ ont été incubés, en duplicat, avec 3.10⁴ cellules-L transfectées avec l'une des molécules HLA-DR ou HLA-DP4, en présence ou en l'absence du peptide approprié. Chaque barre représente le nombre moyen de spots des duplicats ± écart type.

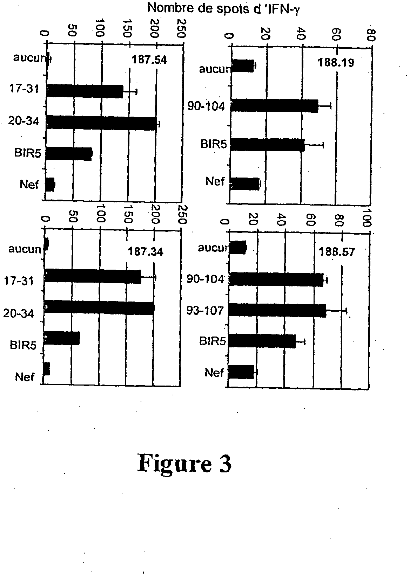

- la figure 3 illustre la présentation de la survivine aux lignées de lymphocytes T CD4+ peptide-spécifique. Des lignées de lymphocytes T CD4+ spécifiques de la survivine ont été obtenues à partir de PBMC de deux donneurs sains (187 et 188). La survivine (BIR5) et la protéine Nef du VIH ont été incubées pendant 4 heures avec des cellules dendritiques autologues immatures. Les cellules dendritiques (CD) ont ensuite été lavées, puis incubées avec les lymphocytes T CD4+ (2.10⁴ CD et 10⁴ lymphocytes T CD4+). La réponse des lymphocytes T CD4+ a été mesurée par ELISPOT IFN-γ. Chaque barre représente le nombre moyen de spots des duplicats ± écart type.

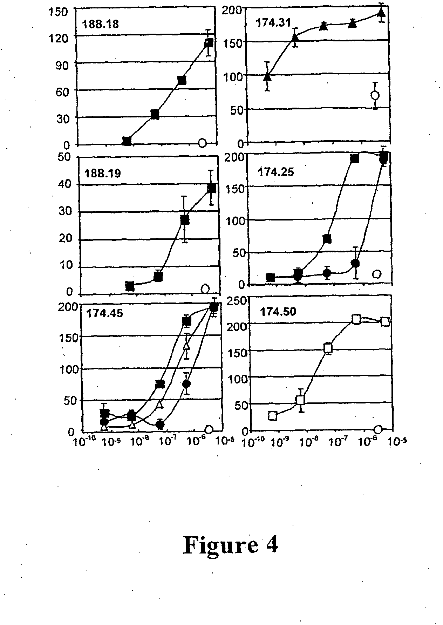

- la figure 4 illustre la réponse des lignées de lymphocytes T CD4+. spécifiques de la survivine à des doses décroissantes de peptide. Des lignées de lymphocytes T CD4+ spécifiques de la survivine ont été obtenues à partir de PBMC de deux donneurs sains (174 et 188). Les lignées ont été incubées avec des concentrations décroissantes (10⁻⁵ à 10⁻¹⁰ M) du peptide 17-31 (▲), 90-104 (■), 93-107 (■), 96-110 (Δ) ou 128-142 (□), en présence de 10⁵ PBMC autologues. La réponse des lymphocytes T CD4+ a été mesurée par ELISPOT IFN-γ.

### EXEMPLE 1 :ACTIVITE DE LIAISON DES PEPTIDES DE LA SURVIVINE VIS-A-VIS DES MOLECULES HLA II

### 1) Matériels et méthodes

### a) Peptides et protéines

Des peptides de 15 acides aminés (15-mères) couvrant la totalité de la séquence de la survivine (SwissProt 015392) ont été sélectionnés en fonction de la présence de résidus aromatiques ou hydrophobes en position 1 à 5, en particulier en position 3 ou 4, pour l'ancrage dans la poche P1 des molécules HLA-DR et HLADP4.

Les séquences des peptides sélectionnés sont présentées dans le Tableau II et la liste de séquences jointe en annexe.

Les peptides ont été synthétisés selon la stratégie Fmoc en synthèse parallèle sur phase solide (synthétiseur 357 MPS, ADVANCED CHEMTECH EUROPE), éventuellement purifiés par RP-HPLC (colonne C₁₈ Vydac, INTERCHIM) et contrôlés par spectrométrie de masse (ES-MS) et HPLC analytique.

La survivine (Bir5 ou BIR5) et la protéine Nef du VIH sont produites dans *E*. *coli,* sous forme de protéines fusion avec la glutathion S-transférase (GST), purifiées sur une colonne de glutathion et séparées de la GST par clivage protéolytique.

**Tableau II: Séquences des peptides de la survivine (SEQ ID NO: 1 à 28)**

| SEQ ID NO : | positions | séquence | | | | | | | | | | | | | | |
|---|---|---|---|---|---|---|---|---|---|---|---|---|---|---|---|---|
| 1 | 1-15* | M | G | A | P | T | L | P | P | A | W | Q | P | F | L | K |
| 2 | 4-18 | P | T | L | P | P | A | W | Q | P | F | L | K | D | H | R |
| 3 | 8-22 | P | A | W | Q | P | F | L | K | D | H | R | 1 | S | T | F |
| 4 | 11-25 | Q | P | F | L | K | D | H | R | I | S | T | F | K | N | W |
| 5 | 17-31 | H | R | I | S | T | F | K | N | W | P | F | L | E | G | C |
| 6 | 19-33 | I | S | T | F | K | N | W | P | F | L | E | G | C | A | C |
| 7 | 20-34 | S | T | F | K | N | W | P | F | L | E | G | C | A | C | T |
| 8 | 23-37 | K | N | W | P | F | L | E | G | C | A | C | T | P | E | R |
| 9 | 36-50 | E | R | M | A | E | A | G | F | I | H | C | P | T | E | N |
| 10 | 41-55 | A | G | F | 1 | H | C | p | T | E | N | E | P | D | L | A |
| 11 | 52-66 | P | D | L | A | Q | C | F | F | C | F | K | E | L | E | G |
| 12 | 56-70 | Q | C | F | F | C | F | K | E | L | E | G | W | E | P | D |
| 13 | 59-73 | F | C | F | K | E | L | E | G | W | E | P | D | D | D | P |
| 14 | 62-76 | K | E | L | E | G | W | E | P | D | D | D | P | I | E | E |
| 15 | 65-79 | E | G | W | E | P | D | D | D | P | I | E | E | H | K | K |
| 16 | 72-86 | D | P | 1 | E | E | H | K | K | H | S | S | G | C | A | F |
| 17 | 84-98 | C | A | F | L | S | V | K | K | Q | F | E | E | L | T | L |
| 18 | 87-101 | L | S | V | K | K | Q | F | E | E | L | T | L | G | E | F |
| 19 | 90-104 | K | K | Q | F | E | E | L | T | L | G | E | F | L | K | L |
| 20 | 91-105 | K | Q | F | E | E | L | T | L | G | E | F | L | K | L | D |
| 21 | 93-107 | F | E | E | L | T | L | G | E | F | L | K | L | D | R | E |
| 22 | 94-108 | E | E | L | T | L | G | E | F | L | K | L | D | R | E | R |
| 23 | 96-110 | L | T | L | G | E | F | L | K | L | D | R | E. | R | A | K |
| 24 | 99-113 | G | E | F | L | K | L | D | R | E | R | A | K | N | K | I |
| 25 | 102-116 | L | K | L | D | R | E | R | A | K | N | K | I | A | K | E |
| 26 | 111-125 | N | K | I | A | K | E | T | N | N | K | K | K | E | F | E |
| 27 | 122-136 | K | E | F | E | E | T | A | K | K | V | R | R | A | I | E |
| 28 | 128-142 | A | K | K | V | R | R | A | I | E | Q | L | A | A | M | D |

| | | | | | | | | | | | | | | | | |
|---|---|---|---|---|---|---|---|---|---|---|---|---|---|---|---|---|
| * les positions sont numérotées en référence à la séquence de la survivine humaine de 142 acides aminés (SwissProt 015392). | | | | | | | | | | | | | | | | |

### b) Test de liaison HLA II/peptide

Les tests de liaison aux molécules HLA II sont des tests de liaison en compétition avec une révélation immuno-enzymatique, tels que décrits dans le Brevet américain US 6,649,166, Texier et al., J. Immunol., 2000, 164, 3177-3184 et Texier et al., Eur. J. Immunol., 2001, 31, 1837-1846, pour les molécules HLA-DR, et dans la Demande Internationale PCT WO 03/040299 et Castelli et al., J. Immunol., 2002, 169, 6928-6934, pour la molécule HLA-DP4. La mise en oeuvre de ces tests pour mesurer l'activité de liaison de peptides issus de différents antigènes, est illustrée dans le brevet américain US 6,649,166 et les Demandes Internationale PCT WO 02/090382, WO 03/040299 et WO 2004/014936.

De manière plus précise, les peptides : HA 306-318 (PKYVKQNTLKLAT, SEQ ID NO: 29), A3 152-166 (EAEQLRAYLDGTGVE, SEQ ID NO: 30), MT 2-16 (AKTIAYDEEARRGLE, SEQ ID NO: 31), B1 21-36 (TERVRLVTRHIYNREE, SEQ ID NO: 32) YKL (AAYAAAKAAALAA, SEQ ID NO: 33), LOL 191-210 (ESWGAVWRIDTPDKLTGPFT, SEQ ID NO: 34), E2/E168 (AGDLLAIETDKATI, SEQ ID NO: 35) et Oxy 271-287 (EKKYFAATQFEPLAARL, SEQ ID NO: 36), biotinylés au niveau du résidu NH₂ terminal, selon le protocole décrit dans Texier et al., J. Immunol., 2000, 164, 3177-3184, sont utilisés comme traceur dans les conditions telles que précisées dans le Tableau ci-après.

**TABLEAU III : Conditions des tests de liaison aux molécules HLA II**

| **Allèles** | **Dilution de HLA II** | **Traceurs** | **Concentration traceur (nM)** | **pH optimal** | **Temps d'incubation (h)** | **IC₅₀ (nM)** |
|---|---|---|---|---|---|---|
| DRB1*0101 | 1/200 | HA 306-318 | 1 | 6 | 24 | 5,5 |
| DRB1*0301 | 1/40 | MT 2-16 | 200 | 4,5 | 72 | 450 |
| DRB1*0401 | 1/80 | HA306-318 | 30 | 6 | 24 | 50 |
| DRB1*0701 | 1/60 | YKL | 10 | 5 | 24 | 15 |
| DRB1*1101 | 1/80 | HA 306-318 | 10 | 5 | 24 | 25 |
| DRB1*1301 | 1/40 | B1 21-36 | 100 | 4,5 | 72 | 800 |
| DRB1*1501 | 1/100 | A3152-166 | 30 | 4,5 | 72 | 52 |
| DRB5*0101 | 1/80 | HA 306-318 | 10 | 5,5 | 24 | 8 |
| DRB3*0101 | 1/40 | Lol 191-120 | 10 | 5,5 | 24 | 14 |
| DRB4*0101 | 1/20 | E2/E168 | 10 | 5 | 72 | 15 |
| DBP1*0401 | 1/80 | Oxy 271-287 | 1 | 5 | 24 | 10 |
| DPB1*0402 | 1/40 | Oxy 271-287 | 1 | 5 | 24 | 6,5 |

Les résultats sont exprimés sous la forme de la concentration (nM) de peptide compétiteur qui inhibe 50% de la liaison maximale du peptide traceur biotinylé (IC₅₀). La sensibilité de chaque test est reflétée par les IC₅₀ observées avec les peptides non-biotinylés qui correspondent aux traceurs; les IC₅₀ des traceurs varient d'un facteur inférieur à trois. Un peptide présentant une activité de liaison (IC₅₀) à une molécule HLA II, inférieure à 1000 nM, possède une bonne affinité pour cette molécule HLA II, un peptide, présentant une activité de liaison (IC₅₀) à une molécule HLA II, inférieure à 100 nM, possède une forte affinité pour cette molécule HLA II.

### 2) Résultats

Trois régions distinctes de la survivine sont capables de se lier avec une bonne affinité (IC₅₀ < 1000 nM) à aux moins trois molécules HLA II prépondérantes, notamment dans la population caucasienne: les régions 17-34 (N-terminale), 84-113 et 122-142 (C-terminale), (Tableau IV).

**Tableau IV : Activités de liaison des peptides de la survivine vis-à-vis des 12 molécules HLA II prépondérantes° (IC₅₀ en nM)**

| **peptides** | **DR1** | **DR3** | **DR4** | **DR7** | **DR11** | **DR13** | **DR15** | **DRB3** | **DRB4** | **DRB5** | **DP401** | **DP402** | **nb de molécules** |
|---|---|---|---|---|---|---|---|---|---|---|---|---|---|
| Référence** | 2 | 346 | 33 | 16 | 10 | 609 | 41 | 16 | 24 | 8 | 15 | 25 | fixées |
| 1-15 | 10800 | >100000 | >100000 | >100000 | **63** | >100000 | 80000 | >100000 | >100000 | 8000 | 9200 | 6928 | 1 |
| 4-18 | 45000 | >100000 | >100000 | >100000 | **33** | 40000 | >100000 | >100000 | >100000 | 5477 | 8998 | 3594 | 1 |
| 8-22 | 6573 | 16000 | 46000 | 9391 | **41** | 2314 | 5200 | 8718 | 29000 | 2191 | 21000 | 10450 | 1 |
| 11-25 | 6000 | 65000 | 45000 | 8000 | **329** | 2408 | **22** | 3674 | 75000 | 2258 | 33000 | 25000 | 2 |
| 17-31 | 19 | >100000 | 9200 | **18** | **247** | **735** | 3 | >100000 | 7500 | **302** | **40** | **29** | **8** |
| 19-33 | 10 | >100000 | **615** | **748** | **147** | 9500 | **81** | >100000 | 3118 | 3779 | **32** | **24** | **7** |
| 20-34 | 11 | >100000 | 5200 | 20000 | **210** | 2449 | 1304 | >100000 | 5500 | 6500 | **210** | **114** | **4** |
| 23-37 | **71** | >100000 | 5200 | 60000 | 37000 | >100000 | >100000 | >100000 | >100000 | 3421 | >100000 | 3700 | 1 |
| 36-50 | 2550 | >100000 | 17000 | 17000 | 5586 | >100000 | >100000 | >100000 | 49000 | 3795 | 3622 | 3169 | 0 |
| 41-55 | 2315 | >100000 | 5000 | 3735 | 1148 | >100000 | 7000 | 10000 | **328** | 3382 | >100000 | >100000 | 1 |
| 52-66 | 4100 | 11832 | 22000 | 6673 | **92** | 23000 | 2245 | 12649 | >100000 | 5348 | >100000 | 4583 | 1 |
| 56-70 | 1597 | >100000 | >100000 | 13000 | **363** | >100000 | 3000 | 12000 | >100000 | 20000 | >100000 | >100000 | 1 |
| 59-73 | 3759 | >100000 | 58000 | 9000 | 1844 | >100000 | 8000 | >100000 | >100000 | 45000 | >100000 | >100000 | 0 |
| 62-76 | >100000 | >100000 | >100000 | >100000 | >100000 | >100000 | 65000 | 29000 | >100000 | >100000 | >100000 | >100000 | 0 |
| 65-79 | >100000 | >100000 | >100000 | >100000 | >100000 | >100000 | >100000 | >100000 | >100000 | >100000 | >100000 | >100000 | 0 |
| 72-86 | 95000 | >100000 | >100000 | >100000 | >100000 | 11000 | >100000 | >100000 | >100000 | 6200 | >100000 | >100000 | 0 |
| 84-98 | 2828 | 2569 | 19000 | **179** | **10** | **846** | **574** | 11832 | 1327 | 1766 | **167** | **113** | **6** |
| 87-101 | **150** | >100000 | 6450 | **705** | 2062 | >100000 | 4796 | 6797 | 9798 | 6000 | 3666 | 6387 | 2 |
| 90-104 | **40** | >100000 | **748** | **140** | 2349 | >100000 | 7500 | 48000 | >100000 | **280** | **48** | **19** | **6** |
| 91-105 | **12** | >100000 | 1587 | **56** | 2353 | >100000 | 3162 | 30000 | >100000 | **145** | **11** | **12** | **5** |
| 93-107 | **128** | >100000 | 5500 | 2793 | **548** | 7800 | **612** | 45000 | >100000 | **529** | **20** | **67** | **6** |
| 94-108 | n.d*. | n.d. | n.d. | n.d. | n.d. | n.d. | n.d. | n.d. | n.d. | n.d. | n.d. | n.d. | 0 |
| 96-110 | **18** | 5477 | 1666 | 5639 | 7 | **302** | **490** | >100000 | 3600 | **103** | 2915 | **620** | **6** |
| 99-113 | **48** | 4673 | 2134 | 6462 | **40** | 9165 | 1200 | >100000 | 22000 | **22** | 40000 | >100000 | 3 |
| 102-116 | 7746 | >100000 | >100000 | 50000 | 8944 | >100000 | >100000 | >100000 | >100000 | **18** | >100000 | >100000 | 1 |
| 111-125 | 5000 | >100000 | >100000 | >100000 | 30000 | 70000 | 90000 | >100000 | 70000 | **16** | >100000 | >100000 | 1 |
| 122-136 | 5657 | 5809 | >100000 | 20000 | **310** | **995** | 1200 | >100000 | >100000 | **447** | >100000 | >100000 | 3 |
| 128-142 | **6** | 12000 | **352** | 1342 | **25** | >100000 | **284** | >100000 | 1803 | 1090 | 13000 | 20000 | **4** |

| | | | | | | | | | | | | | |
|---|---|---|---|---|---|---|---|---|---|---|---|---|---|
| ° les valeurs sont le résultat de 3 expériences indépendantes; elles ne varient pas de plus d'un facteur 3 entre les expériences. * non déterminé (peptide insoluble) ** activité de liaison du traceur | | | | | | | | | | | | | |

En revanche, les peptides issus du reste de la séquence ne présentent pas d'activité de liaison significative pour au moins trois molécules HLA II prépondérantes dans la population caucasienne. En outre, aucun peptide ne se lie aux molécules DR3 et DRB3.

Certains peptide sont spécifiques d'un allèle comme les peptides 1-15, 4-18 et 52-66 qui se lient seulement à la molécule HLA-DR11 et le peptide 41-55 qui se lie seulement à la molécule HLA-DRB4.

Les peptides de la région N-terminale ont une bonne affinité pour les molécules HLA-DR1, DR7, DR11, DR13, DR15, DRB5, DP401 et DP402.

Dans la région 84-113, les capacités de liaison des quatre peptides chevauchants 84-98, 90-104, 93-107 et 96-110 couvrent les molécules HLA-DR1, DR4, DR7, DR11, DR13, DR15, DRB5, DP401 et DP402. Le peptide 91-105 présente une forte affinité pour les molécules DR1, DR7, DRB5, DP401 et DP402.

Dans la région C-terminale, le peptide 128-142 se lie avec une forte affinité aux molécules DR1 et DR11 et une affinité moindre aux molécules HLA-DR4 et HLA-DR15.

Sept peptides présentant une bonne affinité pour aux moins quatre molécules HLA II prépondérantes dans la population caucasienne choisies parmi HLA -DR1, -DR4, -DR7, -DR11, -DR15, DRB5, DP401 et DP402, ont été choisis pour les tests d'immunogénicité : 17-31, 20-34, 84-98, 90-104, 93-107, 96-110 et 128-142.

### EXEMPLE 2 : IMMUNOGENICITE DES PEPTIDES DE LA SURVIVINE IN VITRO

La capacité des peptides ayant une bonne affinité pour les molécules HLA-II prépondérantes dans la population caucasienne, à induire *in vitro* une stimulation de lymphocytes T spécifiques, a été évaluée à partir de prélèvements sanguins d'individus sains (non porteurs de tumeur). Il s'agit d'évaluer la capacité à recruter des lymphocytes précurseurs CD4+ alors qu'ils sont chez un individu naïf à une très faible fréquence, c'est-à-dire d'effectuer une immunisation *in vitro* au moyen de ces peptides.

### 1) Matériels et méthodes

### a) individus testés

Les cellules mononucléées du sang périphérique (PBMC) de sept donneurs sains ont été séparées sur un gradient de Ficoll (Ficoll-Hyperpaque, SIGMA-ALDRICH) puis le génotype HLA-DR et HLA-DP des donneurs a été déterminé par SSP, à l'aide du kit OLERUP SSP™ HLA-DPB1 et HLA-DRB1 (OLERUP SSP AB). Les allèles HLA-DRB1 et HLA-DPB1 de ces individus sont présentés dans le Tableau V.

**Tableau V: Typage HLA des individus testés**

| **Donneur sain** | **DRB1** | **seconde molécule DR** | | | **DPB1** |
|---|---|---|---|---|---|
| **D169** | **DRB1*1502** | | | | **DPB1*0402** |
| **D171** | **DRB1*0101** | **DRB1*0401** | **DRB4** | | **DPB1*0401** |
| **D174** | **DRB1*0704** | **DRB1*1501** | **DRB4** | **DRB5** | **DPB1*0401** |
| **D179** | **DRB1*0701** | **DRB1*1301** | **DRB4** | **DRB3** | |
| **D187** | **DRB1*0701** | **DRB1*0401** | **DRB4** | | |
| **D188** | **DRB1*1101** | | **DRB3** | | **DPB1*0401** |
| **D180** | **DRB1*0101** | **DRB1*1101** | | | **DPB1*0401** |

### b) Obtention de lignées de lymphocytes T CD4+ spécifiques de la survivine et restreintes aux molécules HLA II prépondérantes

Les cellules mononucléées du sang périphérique (PBMC) ont été séparées sur un gradient de Ficoll (Ficoll-Hyperpaque, SIGMA-ALDRICH). Les PBMCs ont ensuite été mises en culture dans du milieu AIM V (LIFE TECHNOLOGIES; 10⁷ cellules /mL) et incubées dans des flasques, à l'étuve à 37°C en présence de 5% CO₂. Après une nuit d'incubation, les cellules non adhérentes ont été récupérées, puis les lymphocytes T CD4+ ont été purifiés à l'aide d'anticorps anti-CD4 couplés à des billes magnétiques (kit MYLTENYI BIOTECH), triés en cytométrie de flux (MACS) selon les recommandations du fabricant (MYLTENYI BIOTECH), et congelés. Les cellules adhérentes ont été incubées pendant 5 à 6 jours, dans du milieu AIM V contenant 1000U/ml de GM-CSF et 1000U/ml d'IL-4 humaines recombinantes (rh-GM-CST et rh-IL-4 ; TEBU), puis les cellules différenciées en cellules dendritiques (cellules dendritiques immatures) ont ensuite été cultivées pendant 2 jours, en présence de 1 µg/ml de LPS (SIGMA), 1000 U/ml de rhIL-4 et 1000 U/ml de rh-GM-CSF, de manière à induire leur maturation.

La qualité des préparations de cellules dendritiques est évaluée par cytométrie de flux (FACScalibur flow cytometer™, BECTON DICKINSON) assistée par le logiciel Cell Quest Pro™ (BECTON DICKINSON). Pour ce faire, les cellules dendritiques sont marquées avec des anticorps anti-CD14, -CD86, -HLA-DR, -CD80 (BECTON DICKINSON), -Cdla, -HLA-ABC, -CD83 et -CD16 (BECKMAN COULTER), conjugués à un fluorochrome.

Les cellules dendritiques matures (CD ; 5.10⁵ cellules dans 1 mL) ainsi obtenues ont été incubées avec un mélange de peptides (10 µg de chaque peptide dans du milieu IMDM (INVITROGEN) supplémenté avec de la glutamine (24 mM, SIGMA), de l'asparagine (55 mM, SIGMA), de l'arginine (150 mM, SIGMA), de la pénicilline (50UI/ml, INVITROGEN), de la streptomycine (50 mg/ml, INVITROGEN) et 10 % de sérum humain, dénommé ci-après milieu IMDM complet, pendant 4 heures à 37°C. Les cellules dendritiques matures ont ensuite été lavées puis incubées, en présence de lymphocytes T CD4+ autologues préalablement décongelés (10⁴ CD et 10⁵ T CD4+), dans 200 µL de milieu IMDM complet contenant 1000 U/ml d'IL-6 (R&D systems) et 10 ng/ml d'IL-12 (R&D systems). Après 7 jours (J7), la culture a été stimulée une première fois, au moyen de cellules dendritiques matures préalablement décongelées et chargées par le mélange de peptides, dans du milieu contenant de l'IL-2 (10 U/ml) et de l'IL-7 (5 ng/ml). Après deux stimulations supplémentaires (J14 et J21) au moyen de cellules dendritiques chargées, dans les mêmes conditions, la spécificité des cellules pour les peptides a été analysée à J28 et J29, par mesure de la production d'IFN-γ en ELISPOT. c) Obtention de lignées de lymphocytes T CD4+ spécifiques de la survivine et

### restreintes à HLA-DP4

Des lignées de lymphocytes T CD4+ spécifiques de la survivine et restreintes à HLA-DP4 ont été produites en utilisant le mélange des peptides 84-98, 93-107, 90-104 et 19-33 pour la stimulation des cellules dendritiques autologues, selon un protocole similaire à celui utilisé pour la production de lignées de lymphocytes T restreintes à l'ensemble des molécules HLA-DR prépondérantes dans là population caucasienne.

### d) Analyse de la spécificité des lignées en ELISPOT

L'anticorps monoclonal humain anti-IFN-γ 1-D1K (MABTECH), dilué à 10 µg/ml dans du tampon PBS a été adsorbé sur des plaques de nitrocellulose (Multiscreen HA ; MILLIPORE) pendant 1 heure à 37°C. Les plaques ont ensuite été lavées avec du PBS puis saturées avec du milieu IMDM complet (100 µl/puits), pendant 1 h à 37 °C. Les cellules présentatrices d'antigène sont des PBMC autologues, des cellules dendritiques autologues immatures préparées comme précisé ci-dessus ou une lignée de fibroblastes de souris (lignée L), transfectée avec l'ADNc codant pour l'une des molécules HLA-DR ou HLA-DP4 à tester (Yu et al. Hum. Immunol., 1990, 27, 132-135), de façon à vérifier la spécificité des lignées vis-à-vis des molécules HLA-DR et HLA-DP4. Les cellules présentatrices d'antigène (10⁵ PBMC autologues, 3.10⁴ cellules-L transfectées ou 2.10⁴ cellules dendritiques autologues immatures) et les lymphocytes T CD4+ à tester (10⁴ lymphocytes T CD4⁺) ont ensuite été ajoutés dans les plaques et incubées 24 h à 37 °C, en présence ou en l'absence d'un seul peptide (2 µg) d'un mélange de peptides (2 µg de chaque peptide) ou d'une protéine (Bir5 ou Nef). Les protéines (1 µM) sont incubées 4 h à 37° C en présence de cellules dendritiques immatures, puis lavées. Les peptides sont ajoutés directement sur les plaques. Pour les analyses dose-réponse, les peptides sont utilisés à différentes concentration variant de 10⁻⁵ à 10⁻¹⁰ M. Après trois lavages successifs avec de l'eau, puis avec du tampon PBS Tween 0,05%, et enfin avec du PBS seul, 100 µl d'anticorps secondaire anti-IFN-γ conjugué à la biotine (7-B6-1-biotin, MABTECH), dilué à 0,25 µg/ml dans du PBS contenant 1% BSA, a été ajouté dans chaque puits. Après une heure d'incubation, les plaques ont été lavées à nouveau et incubées avec 100 µl/puits d'Extravidin-phosphatase (E-2636, SIGMA,), diluée au 1/6000. Après lavage des plaques en tampon PBS, 100 µl de substrat NBT/BCIP (B-5655, SIGMA) dilué dans de l'eau (1 tablette dans 10 ml d'eau) ont été distribués dans chaque puits. La révélation immunoenzymatique a été arrêtée après environ 10 minutes, par rinçage extensif des plaques dans l'eau, et les spots colorés ont été comptés à l'aide d'un lecteur automatique (Elispot Reader System, AID). Les lignées sont considérées positives lorsque le nombre de spots est supérieur à trois fois celui obtenu avec le témoin négatif (témoin sans peptides) avec un minimum de 50 spots. Le témoin sans cellules présentatrices permet de vérifier la spécificité de la réponse pour HLA-DR ou HLA-DP4 (témoin de restriction).

### 2) Résultats

### a) Lignées spécifiques de l'ensemble des molécules HLA II prépondérantes

98 lignées de lymphocytes T CD4+ spécifiques de la survivine ont été obtenues à partir de sept donneurs sains (Tableau VI). La spécificité des lignées pour les peptides de la survivine a été analysée par ELISPOT IFN-γ, en utilisant des PBMC autologues comme cellules présentatrices d'antigène.

**Tableau VI : Résumé de la spécificité des lignées de lymphocytes T**

| **Nb de lignées de lymphocytes T** | | | | | | | |
|---|---|---|---|---|---|---|---|
| **Individu** | **peptide** | | | | | | |
| | **17-31** | **20-34** | **84-98** | **90-104** | **93-107** | **96-110** | **128-142** |
| **D169** | 0 | 0 | 0 | 1 | 1 | 12 | 0 |
| **D171** | 2 | 1 | 3 | 3 | 0 | 0 | 7 |
| **D174** | 10 | 0 | 0 | 3 | 2 | 1 | 1 |
| **D179** | 1 | 0 | 2 | 2 | 0 | 2 | 4 |
| **D187** | 4 | 6 | 0 | 1 | 0 | 2 | 1 |
| **D188** | 1 | 2 | 3 | 7 | 3 | 1 | 0 |
| **D180** | 0 | 0 | 3 | 2 | 2 | 1 | 1 |
| **total** | 18 | 9 | 11 | 19 | 8 | 19 | 14 |
| **Fréquence de répondeurs** | 5/7 | 3/7 | 4/7 | 7/7 | 4/7 | 6/7 | 5/7 |

La figure 1 montre les résultats obtenus avec 12 lignées de lymphocytes T CD4+ dérivées des donneurs sains 171 et 174. Ces lignées sont stimulées par le mélange de peptides présenté par des cellules dendritiques autologues mais pas en l'absence du mélange. Parmi les 60 puits ensemencés pour chaque donneur, 12 et 13 lignées de lymphocytes T peptide-spécifique ont été obtenues, respectivement pour le donneur 171 et le donneur 174, suggérant que ces lignées dérivent de quelques précurseurs de lymphocytes T qui ont été amplifiés par les stimulations hebdomadaires avec les peptides. De fait, les lignées de lymphocytes T sont généralement spécifiques d'un seul peptide ou de deux peptides chevauchants, suggérant qu'elles sont spécifiques de la région commune aux deux peptides (figure 1). Tous les peptides ont induit au moins une lignée (Tableau VI et figure 1).

Les sept donneurs couvrent les haplotypes HLA-DR prépondérants dans la population (Tableau VI), à savoir : HLA-DR1, -DR4, -DR7, -DR1, -DR13 et-DR15 et la deuxième molécule DR correspondante (DRB3, DRB4 et DRB5). Parmi les molécules HLA-DR prépondérantes dans la population caucasienne, seule la molécule HLA-DR3 est absente. Cinq donneurs possèdent une molécule HLA-DP4, en accord avec la fréquence de cette molécule dans la population caucasienne. Le mélange de peptides induit des lignées de lymphocytes T CD4+ spécifiques chez chaque donneur, bien que l'échantillonnage de donneur ait été choisi de façon à inclure de multiples haplotypes HLA II. Le peptide 17-31 induit 18 lignées de lymphocytes T, le peptide 20-34, 9 lignées, le peptide 84-98, 11 lignées, le peptide 90-104, 19 lignées, le peptide 93-107, 8 lignées, le peptide 96-110, 19 lignées et le peptide 128-142, 14 lignées. Le peptide 90-104 est immunogène chez tous les donneurs et les peptides 17-31, 96-110 et 128-142 ont induit des lignées de lymphocytes T chez cinq ou six des sept donneurs.

Les éléments de restriction des molécules HLA II responsables de la stimulation spécifique des lymphocytes T CD4+ par les peptides a été évaluée pour 19 lignées différentes de lymphocytes T CD4+, par ELISPOT IFN-γ, en présence de cellules-L transfectées par l'une des molécules HLA-DR ou par la molécule HLA-DP4, ou en l'absence de cellules présentatrices. Les lignées sont induites spécifiquement par la présentation du peptide par les cellules présentatrices d'antigène étant donné qu'aucune production d'IFN-γ n'est détectée en l'absence de peptide (Figure 2) et qu'une stimulation modeste est observée en l'absence de cellules présentatrices.

La figure 2 illustre les résultats obtenus pour 12 des lignées testées. La plupart des lignées de lymphocytes T sont stimulées par leur peptide présenté par une seule molécule HLA II. Cependant, une dégénérescence de la reconnaissance est observée, par exemple pour les lignées 174.31 et 187.34. La stimulation ne résulte pas de la présentation des peptides par des lymphocytes T voisins activés, dans la mesure où seule une stimulation modeste est observée en l'absence de cellules-L. Ces résultats montrent que DR7, DR15, DP4 et DRB4 sont des éléments de restriction pour le peptide 17-31 ; DP4 pour le peptide 20-34, DR7, DR11, DRB5 et DP4 pour le peptide 90-104, DR11 et DP4 pour le peptide 93-107, DR4, DR7 et DRB4 pour le peptide 96-110 et DR15 pour le peptide 128-142.

La capacité de sept lignées de lymphocytes T peptide-spécifique dérivées de deux donneurs différents, à reconnaître la protéine native a également été analysée. Les résultats présentés à la figure 3 sont représentatifs de ces expériences. Les deux lignées de lymphocytes T (187.54 et 187.34) spécifiques des peptides 17-31 et 20-34 reconnaissent spécifiquement les cellules dendritiques autologues, préalablement chargées avec la survivine recombinante. Elles ne sont pas stimulées en présence de cellules dendritiques non-chargées ou chargées par la protéine Nef du VIH. De même, les lignées de lymphocytes T 188.19 et 188.57 sont stimulées par les peptides 90-104 et 93-107, seuls ou en mélange, et reconnaissent spécifiquement la protéine survivine.

Enfin, la sensibilité de la reconnaissance du peptide par les lymphocytes T a été évaluée, en présence de doses décroissantes de peptide (Figure 4). Mis à part le peptide 93-107, tous les peptides stimulent les lymphocytes T et sont reconnus de façon efficace par les lignées de lymphocytes T peptide-spécifique, à des concentration inférieures à 10⁻⁶M. Le peptide 17-31 est particulièrement efficace puisqu'il est capable de stimuler des lymphocytes T CD4+ autologues à une très faible concentration (10⁻¹⁰ M).

### b) Lignées spécifiques d'HLA-DP4

4 lignées spécifiques d'un peptide de la survivine et restreintes à HLA-DP4 ont été mises en évidence ; ces lignées sont stimulées par le mélange de peptides présenté par HLA-DP4 mais pas en l'absence du mélange (témoin négatif) ou d'HLA-DP4 (témoin sans APC, Tableau VII).

**Tableau VII: Immunogénicité des peptides dans un contexte HLA-DP4**

| **Nombre de spots** | | | | | | | | |
|---|---|---|---|---|---|---|---|---|
| **donneur** | **lignées** | **témoin négatif** | **mélange de peptides** | **84-98** | **93-107** | **90-104** | **19-33** | **sans APC** |
| 169 | 169-14 | 2(±0) | 210(±11) | 4(±2) | 3(±1) | 182(±21) | 1(±0) | 56(±21) |
| | 169-34 | 4(±4) | 255(±18) | 3(±3) | 2(±2) | 4(±2) | 228(±17) | 10(±3) |
| | 169-56 | 3(±1) | 199(±24) | 2(±2) | 2(±2) | 2(±1) | 195(±0) | 104(±30) |
| 171 | 171-3 | 5(±4) | 103(±18) | 7(±1) | 145(±4) | 62(±33) | 5(±0) | 1(±1) |

Chaque lignée répond à un des peptides du mélange. Sur les quatre peptides du mélange, trois sont immunogènes dans un contexte HLA-DP4 (peptides 93-107, 90-104 et 19-33).

Ainsi que cela ressort de ce qui précède, l'invention ne se limite nullement à ceux de ses modes de mise en oeuvre, de réalisation et d'application qui viennent d'être décrits de façon plus explicite .

## Revendications

1. Peptide dérivé de l'isoforme alpha de la survivine pour utilisation comme antigène dans la vaccination prophylactique ou thérapeutique du cancer, lequel peptide étant sélectionné dans le groupe constitué par :
a) les peptides de 13 à 18 acides aminés consécutifs situés entre les positions 17 et 34 de l'isoforme alpha de la survivine,
b) les peptides de 13 à 30 acides aminés consécutifs situés entre les positions 84 et 113 de l'isoforme alpha de la survivine, et
c) les peptides de 13 à 21 acides aminés consécutifs situés entre les positions 122 et 142 de l'isoforme alpha de la survivine,
lesquels peptides en a), b) et c) se lient avec une activité de liaison inférieure à 1000 nM à au moins quatre molécules HLA-II prépondérantes dans la population caucasienne sélectionnées dans le groupe constitué par les molécules HLA-DR1, HLA-DR3, HLA-DR4, HLA-DR7, HLA-DR11, HLA-DR13, HLA-DR15, HLA-DRB3, HLA-DRB4, HLA-DRB5 et HLA-DP4 codées respectivement par les allèles HLA DRB1*0101, DRB1*0301, DRB1*0401, DRB1*0701, DRB1*1101, DRB1*1301, DRB1*1501, DRB3*0101, DRB4*0101, DRB5*0101, DP*0401 et DP*0402, l'activité de liaison correspondant à la concentration dudit peptide qui inhibe 50 % de la liaison maximale d'un traceur biotinylé dans un test de liaison molécule HLA II/peptide en compétition, réalisé dans les conditions telles que précisées dans le Tableau ci-dessous pour chaque allèle HLA II :
| **Allèles** | **Traceurs (SEQ ID NO :)** | **Concentration traceur (nM)** | **pH optimal** | **Temps d'incubation (h)** |
|---|---|---|---|---|
| DRB1*0101 | 29 | 1 | 6 | 24 |
| DRB1*0301 | 31 | 200 | 4,5 | 72 |
| DRB1*0401 | 29 | 30 | 6 | 24 |
| DRB1*0701 | 33 | 10 | 5 | 24 |
| DRB1*1101 | 29 | 10 | 5 | 24 |
| DRB1*1301 | 32 | 100 | 4,5 | 72 |
| DRB1*1501 | 30 | 30 | 4,5 | 72 |
| DRB5*0101 | 29 | 10 | 5,5 | 24 |
| DRB3*0101 | 34 | 10 | 5,5 | 24 |
| DRB4*0101 | 35 | 10 | 5 | 72 |
| DBP1*0401 | 36 | 1 | 5 | 24 |
| DPB1*0402 | 36 | 1 | 5 | 24 |
et lesquels peptides en a), b) et c) sont capables d'induire des lymphocytes T CD4+ spécifiques de la survivine.

2. Peptide pour utilisation comme antigène dans la vaccination prophylactique ou thérapeutique du cancer selon la revendication 1, **caractérisé en ce que** dit peptide est apte à être présenté par au moins une molécule HLA-DP401 ou HLA-DP402.

3. Peptide pour utilisation comme antigène dans la vaccination prophylactique ou thérapeutique du cancer selon la revendication 1 ou 2 , **caractérisé en ce qu'**il est sélectionné dans le groupe constitué par les peptides : 17-31, 19-33, 20-34, 84-98, 90-104, 91-105, 93-107, 96-110 et 128-142.

4. Peptide pour utilisation comme antigène dans la vaccination prophylactique ou thérapeutique du cancer selon la revendication 3, **caractérisé en ce que** ledit peptide est apte à être présenté par une molécule HLA-DP401 ou HLA-DP402 et **en ce qu'**il est sélectionné dans le groupe constitué par les peptides : 17-31, 19-33, 20-34, 84-98, 90-104, 91-105, 93-107 et 96-110.

5. Peptide pour utilisation comme antigène dans la vaccination prophylactique ou thérapeutique du cancer selon la revendication 3 ou 4-, **caractérisé en ce que** ledit peptide est sélectionné dans le groupe constitué par les séquences SEQ ID NO : 5, 6, 7, 17, 19, 20, 21, 23 et 28.

6. Peptide pour utilisation comme antigène dans la vaccination prophylactique ou thérapeutique du cancer selon l'une quelconque des revendications 1 à 5, **caractérisé en ce que** ledit peptide présente une séquence de 13, 14, 15, 16, 17, 18, 19, 20, 21, 22, 23, 24 ou 25 acides aminés.

7. Peptide pour utilisation comme antigène dans la vaccination prophylactique ou thérapeutique du cancer selon l'une quelconque des revendications 1 à 6, **caractérisé en ce que** ledit peptide est marqué ou complexé.

8. Peptide pour utilisation comme antigène dans la vaccination prophylactique ou thérapeutique du cancer selon la revendication 7, **caractérisé en ce que** ledit peptide est complexé à une molécule HLA II marquée.

9. Fragment polyépitopique comprenant la concaténation d'au moins deux épitopes identiques ou différents dont l'un au moins est un épitope T CD4+ de la survivine inclus dans un peptide tel que défini à l'une quelconque des revendications 1 à 6, pour utilisation comme antigène dans la vaccination prophylactique ou thérapeutique du cancer.

10. Fragment polyépitopique pour utilisation comme antigène dans la vaccination prophylactique ou thérapeutique du cancer selon la revendication 9, **caractérisé en ce que** ledit fragment polyépitopique comprend la concaténation d'au moins un épitope T CD4+ de la survivine inclus dans un peptide tel que défini à l'une quelconque des revendications 1 à 6 et d'au moins un épitope T CD8+ de la survivine et/ou un épitope T CD4+ universel.

11. Fragment polyépitopique pour utilisation comme antigène selon la revendication 10, **caractérisé en ce que** l'épitope T CD8+ est choisi parmi les séquences SEQ ID NO : 37 à 39.

12. Protéine de fusion constituée par une protéine ou un fragment de protéine, fusionné à un peptide tel que défini à l'une quelconque des revendications 1 à 6 ou un fragment polyépitopique tel que défini à l'une quelconque des revendications 9 à 11, pour utilisation comme antigène dans la vaccination prophylactique ou thérapeutique du cancer.

13. Protéine de fusion pour utilisation comme antigène dans la vaccination prophylactique ou thérapeutique du cancer selon la revendication 12, **caractérisée en ce que** ladite protéine ou ledit fragment est sélectionné dans le groupe constitué par une chaîne alpha ou béta d'une molécule HLA II, un fragment de ladite chaîne correspondant à une molécule HLA II soluble et une séquence de ciblage dans l'endosome.

14. Protéine de fusion pour utilisation comme antigène dans la vaccination prophylactique ou thérapeutique du cancer selon la revendication 13, **caractérisée en ce que** ladite séquence de ciblage est dérivée de la chaîne invariable Ii ou de la protéine LAMP-1.

15. Lipopeptide obtenu par addition d'un lipide sur une fonction α-aminée ou une fonction réactive de la chaîne latérale d'un peptide tel que défini à l'une quelconque des revendications 1 à 6 ou d'un fragment polyépitopique tel que défini à l'une quelconque des revendications 9 à 11, pour utilisation comme antigène dans la vaccination prophylactique ou thérapeutique du cancer.

16. Vecteur d'expression comprenant un polynucléotide codant pour un peptide tel que défini à l'une quelconque des revendications 1 à 6, un fragment polyépitopique tel que défini à l'une quelconque des revendications 9 à 11 ou une protéine de fusion telle que définie à l'une quelconque des revendications 12 à 14, sous le contrôle de séquences régulatrices appropriées, pour utilisation comme antigène dans la vaccination prophylactique ou thérapeutique du cancer.

17. Composition immunogène ou vaccinale, **caractérisée en ce qu'**elle comprend au moins un peptide selon l'une quelconque des revendications 1 à 8, un fragment polyépitopique selon la revendication 10 ou 11, une protéine de fusion selon l'une quelconque des revendications 12 à 14, un lipopeptide selon la revendication 15 ou un vecteur selon la revendication 16, et un véhicule pharmaceutiquement acceptable, une substance porteuse ou un adjuvant.

18. Composition immunogène ou vaccinale selon la revendication 17, **caractérisée en ce qu'**elle comprend au moins un peptide selon l'une quelconque des revendications 1 à 8 et un peptide incluant un épitope T CD8+ de la survivine, sous la forme d'un mélange de peptides, d'un fragment polyépitopique et/ou d'un vecteur d'expression codant lesdits peptides ou ledit fragment.

19. Composition immunogène ou vaccinale selon la revendication 18, **caractérisée en ce que** ledit épitope T CD8+ est choisi parmi les séquences SEQ ID NO : 37 à 39.

20. Composition immunogène ou vaccinale selon l'une quelconque des revendications 17 à 19, **caractérisée en ce qu'**elle comprend au moins deux peptides différents tels que définis à l'une quelconque des revendications 1 à 8, sélectionnés dans le groupe constitué par l'une des combinaisons suivantes:
- le peptide 17-31 et l'un au moins des peptides 19-33, 90-104 ou 128-142
- le peptide 19-33 et le peptide 96-110,
- le peptide 90-104 et le peptide 17-31,
- le peptide 96-110 et le peptide 90-104, et
- les peptides 93-107 et 128-142, et l'un au moins des peptides 17-31, 19-33, 96-110 ou 90-104.

21. Composition immunogène selon l'une quelconque des revendications 17 à 20, **caractérisée en ce qu'**elle comprend un épitope T CD4+ universel.

22. Méthode *in vitro* de diagnostic, d'évaluation du pronostic ou de suivi du traitement d'un cancer chez un individu, **caractérisée en ce qu'**elle comprend :
- la mise en contact, *in vitro,* d'un échantillon biologique dudit individu avec un peptide selon l'une quelconque des revendications 1 à 8, et
- la détection de lymphocytes T CD4+ spécifiques de la survivine, par tout moyen approprié.

23. Procédé de tri de lymphocytes T CD4+ spécifiques de la survivine, **caractérisé en ce qu'**il comprend au moins les étapes suivantes :
- la mise en contact, *in vitro,* d'un échantillon cellulaire avec des complexes multimériques HLA II/peptide marqués, formés par la liaison de molécules HLA II solubles avec au moins un peptide selon l'une quelconque des revendications 1 à 8, et
- le tri des cellules liées auxdits complexes HLA II/peptide.

24. Peptide, **caractérisé en ce qu'**il s'agit d'un peptide tel que défini à l'une quelconque des revendications 1 à 8, à l'exclusion du peptide situé entre les positions 89 à 101 de l'isoforme alpha de la survivine.

25. Fragment polyépitopique comprenant la concaténation d'au moins deux épitopes identiques ou différents dont au moins un épitope T CD4+ de la survivine inclus dans un peptide tel que défini à l'une quelconque des revendications 1 à 8 et au moins un épitope T CD8+ de la survivine et/ou un épitope T CD4+ universel.

26. Protéine de fusion constituée par une protéine ou un fragment de protéine, fusionné à un peptide tel que défini à l'une quelconque des revendications 1 à 8 ou un fragment polyépitopique selon la revendication 25.

27. Lipopeptide obtenu par addition d'un lipide sur une fonction α-aminée ou une fonction réactive de la chaîne latérale d'un peptide tel que défini à l'une quelconque des revendications 1 à 8 ou d'un fragment polyépitopique selon la revendication 25.

28. Polynucléotide codant pour un peptide selon la revendication 24, un fragment polyépitopique selon la revendication 25 ou une protéine de fusion selon la revendication 26.

29. Vecteur d'expression comprenant un polynucléotide selon la revendication 28 sous le contrôle de séquences régulatrices appropriées, de la transcription et éventuellement de la traduction.

30. Cellule hôte modifiée par un polynucléotide selon la revendication 28 ou un vecteur d'expression selon la revendication 29.

31. Kit de diagnostic d'évaluation du pronostic ou de suivi du traitement du cancer, **caractérisé en ce qu'**il comprend un peptide selon la revendication 24.

## Patentansprüche

1. Peptid, das von der Isoform alpha des Survivin stammt, zur Verwendung als Antigen bei der prophylaktischen oder therapeutischen Impfung gegen Krebs, wobei das Peptid ausgewählt ist aus der Gruppe, bestehend aus:
a) den Peptiden aus 13 bis 18 fortlaufenden Aminosäuren, die sich zwischen den Positionen 17 und 34 der Isoform alpha des Survivins befinden,
b) den Peptiden aus 13 bis 30 fortlaufenden Aminosäuren, die sich zwischen den Positionen 84 und 113 der Isoform alpha des Survivins befinden, und
c) den Peptiden aus 13 bis 21 fortlaufenden Aminosäuren, die sich zwischen den Positionen 122 und 142 der Isoform alpha des Survivins befinden,
wobei die Peptide in a), b) und c) sich mit einer Bindungsaffinität von unter 1000 nM an wenigstens vier HLA-II-Moleküle binden, die in der kaukasischen Bevölkerung vorherrschend sind, die ausgewählt sind aus der Gruppe, bestehend aus den Molekülen HLA-DR1, HLA-DR3, HLA-DR4, HLA-DR7, HLA-DR11, HLA-DR13, HLA-DR15, HLA-DRB3, HLA-DRB4, HLA-DRB5 und HLA-DP4, die durch die Allele HLA DRB1*0101, DRB1*0301, DRB1*0401, DRB1*0701, DRB1*1101, DRB1*1301, DRB1*1501, DRB3*0101, DRB4*0101, DRB5*0101, DP*0401 bzw. DP*0402 codiert werden, wobei die Bindungsaktivität der Konzentration des Peptids entspricht, die 50% der maximalen Bindung eines biotinylierten Tracer in einem Bindungstest von HLA-II-Molekül/Peptid in Kompetition zu inhibieren, der unter Bedingungen durchgeführt wird, wie sie in der Tabelle unten für jedes HLA-II-Allel präzisiert sind:
| **Allele** | **Tracer (SEQ ID NO:)** | **Konzentration des Tracers (nM)** | **optimaler pH** | **Inkubationszeit (h)** |
|---|---|---|---|---|
| DRB1*0101 | 29 | 1 | 6 | 24 |
| DRB1*0301 | 31 | 200 | 4,5 | 72 |
| DRB1*0401 | 29 | 30 | 6 | 24 |
| DRB1*0701 | 33 | 10 | 5 | 24 |
| DRB1*1101 | 29 | 10 | 5 | 24 |
| DRB1*1301 | 32 | 100 | 4,5 | 72 |
| DRB1*1501 | 30 | 30 | 4,5 | 72 |
| DRB5*0101 | 29 | 10 | 5,5 | 24 |
| DRB3*0101 | 34 | 10 | 5,5 | 24 |
| DRB4*0101 | 35 | 10 | 5 | 72 |
| DBP1*0401 | 36 | 1 | 5 | 24 |
| DPB1*0402 | 36 | 1 | 5 | 24 |
und wobei die Peptide a), b) und c) fähig sind, CD4+T-Lymphozyten, die für Survivin spezifisch sind, zu induzieren.

2. Peptid für eine Verwendung als Antigen bei der prophylaktischen oder therapeutischen Impfung gegen Krebs gemäß Anspruch 1, **dadurch gekennzeichnet, dass** das Peptid geeignet ist, durch wenigstens ein Molekül, HLA-DP401 oder HLA-DP402, präsentiert zu werden.

3. Peptid für eine Verwendung als Antigen bei der prophylaktischen oder therapeutischen Impfung gegen Krebs gemäß Anspruch 1 oder 2, **dadurch gekennzeichnet, dass** es aus der Gruppe, bestehend aus den Peptiden: 17-31, 19-33, 20-34, 84-98, 90-104, 91-105, 93-107, 96-110 und 128-142, ausgewählt ist.

4. Peptid für eine Verwendung als Antigen bei der prophylaktischen oder therapeutischen Impfung gegen Krebs gemäß Anspruch 3, **dadurch gekennzeichnet, dass** das Peptid geeignet ist, durch ein HLA-DP401- oder HLA-DP402-Molekül präsentiert zu werden, und dass es aus der Gruppe, bestehend aus den Peptiden: 17-31, 19-33, 20-34, 84-98, 90-104, 91-105, 93-107 und 96-110, ausgewählt ist.

5. Peptid für eine Verwendung als Antigen bei der prophylaktischen oder therapeutischen Impfung gegen Krebs gemäß Anspruch 3 oder 4, **dadurch gekennzeichnet, dass** das Peptid aus der Gruppe, bestehend aus den Sequenzen SEQ ID NO: 5, 6, 7, 17, 19, 20, 21, 23 und 28, ausgewählt ist.

6. Peptid für eine Verwendung als Antigen bei der prophylaktischen oder therapeutischen Impfung gegen Krebs gemäß einem der Ansprüche 1 bis 5, **dadurch gekennzeichnet, dass** das Peptid eine Sequenz von 13, 14, 15, 16, 17, 18, 19, 20, 21, 22, 23, 24 oder 25 Aminosäuren aufweist.

7. Peptid für eine Verwendung als Antigen bei der prophylaktischen oder therapeutischen Impfung gegen Krebs gemäß einem der Ansprüche 1 bis 6, **dadurch gekennzeichnet, dass** das Peptid markiert oder komplexiert ist.

8. Peptid für eine Verwendung als Antigen bei der prophylaktischen oder therapeutischen Impfung gegen Krebs gemäß Anspruch 7, **dadurch gekennzeichnet, dass** das Peptid an ein markiertes HLA-II-Molekül komplexiert ist.

9. Mehrepitopenfragment, umfassend die Kettenbildung von wenigstens zwei gleichen oder verschiedenen Epitopen, von denen wenigstens eins ein CD4+T-Epitop des Survivins ist, das in einem Peptid, wie es in einem der Ansprüche 1 bis 6 definiert ist, enthalten ist, für eine Verwendung als Antigen bei der prophylaktischen oder therapeutischen Impfung gegen Krebs.

10. Mehrepitopenfragment für eine Verwendung als Antigen bei der prophylaktischen oder therapeutischen Impfung gegen Krebs gemäß Anspruch 9, **dadurch gekennzeichnet, dass** das Mehrepitopenfragment die Verkettung wenigstens eines CD4+T-Epitops des Survivins, eingeschlossen in einem Peptid, wie es in einem der Ansprüche 1 bis 6 definiert ist, und wenigstens eines CD8+T-Epitops des Survivins und/oder eines universellen CD4+T-Epitops umfasst.

11. Mehrepitopenfragment für eine Verwendung als Antigen gemäß Anspruch 10, **dadurch gekennzeichnet, dass** das CD8+T-Epitop aus den Sequenzen SEQ ID NO: 37 bis 39 ausgewählt ist.

12. Fusionsprotein, das durch ein Protein oder ein Proteinfragment fusioniert an ein Peptid, wie es in einem der Ansprüche 1 bis 6 definiert ist, oder ein Mehrepitopenfragment, wie es in einem der Ansprüche 9 bis 11 definiert ist, gebildet ist, für eine Verwendung als Antigen bei der prophylaktischen oder therapeutischen Impfung gegen Krebs.

13. Fusionsprotein für eine Verwendung als Antigen bei der prophylaktischen oder therapeutischen Impfung gegen Krebs gemäß Anspruch 12, **dadurch gekennzeichnet, dass** das Protein oder das Fragment aus der Gruppe ausgewählt ist, die aus einer alpha- oder beta-Kette eines HLA-II-Moleküls, einem Fragment der genannten Kette, das einem löslichen HLA-II-Molekül entspricht, und einer Targetsequenz im Endosom besteht.

14. Fusionsprotein für eine Verwendung als Antigen bei der prophylaktischen oder therapeutischen Impfung gegen Krebs gemäß Anspruch 13, **dadurch gekennzeichnet, dass** die Targetsequenz von der invariablen Kette Ii oder dem Protein LAMP-1 abgeleitet ist.

15. Lipopeptid, erhalten durch Addition eines Lipids an eine α-Aminofunktion oder eine reaktive Funktion der Seitenkette eines Peptids, wie es in einem der Ansprüche 1 bis 6 definiert ist, oder eines Mehrepitopenfragments, wie es in einem der Ansprüche 9 bis 11 definiert ist, für eine Verwendung als Antigen bei der prophylaktischen oder therapeutischen Impfung gegen Krebs.

16. Expressionsvektor, umfassend ein Polynucleotid, das für ein Peptid, wie es in einem der Ansprüche 1 bis 6 definiert ist, ein Mehrepitopenfragment, wie es in einem der Ansprüche 9 bis 11 definiert ist, oder ein Fusionsprotein, wie es in einem der Ansprüche 12 bis 14 definiert ist, codiert, unter der Kontrolle von geeigneten regulatorischen Sequenzen, für eine Verwendung als Antigen bei der prophylaktischen oder therapeutischen Impfung gegen Krebs.

17. Immunogene oder vakzinale Zusammensetzung, **dadurch gekennzeichnet, dass** sie wenigstens ein Peptid gemäß einem der Ansprüche 1 bis 8, ein Mehrepitopenfragment gemäß Anspruch 10 oder 11, ein Fusionsprotein gemäß einem der Ansprüche 12 bis 14, ein Lipopeptid gemäß Anspruch 15 oder einen Vektor gemäß Anspruch 16 und ein pharmazeutisch annehmbares Vehikel, eine Trägersubstanz oder ein Adjuvans umfasst.

18. Immunogene oder vakzinale Zusammensetzung gemäß Anspruch 17, **dadurch gekennzeichnet, dass** sie wenigstens ein Peptid gemäß einem der Ansprüche 1 bis 8 und ein Peptid, umfassend ein CD8+T-Epitop des Survivins, in der Form eines Peptidgemisches, eines Mehrepitopenfragments und/oder eines Expressionsvektors, der die Peptide oder das Fragment codiert, umfasst.

19. Immunogene oder vakzinale Zusammensetzung gemäß Anspruch 18, **dadurch gekennzeichnet, dass** das CD8+T-Epitop aus den Sequenzen SEQ ID NO: 37 bis 39 ausgewählt ist.

20. Immunogene oder vakzinale Zusammensetzung gemäß einem der Ansprüche 17 bis 19, **dadurch gekennzeichnet, dass** sie wenigstens zwei unterschiedliche Peptide, wie sie in einem der Ansprüche 1 bis 8 definiert sind, umfasst, die aus der Gruppe, bestehend aus einer der folgenden Kombinationen:
- Peptid 17-31 und wenigstens eins der Peptide 19-33, 90-104 oder 128-142,
- Peptid 19-33 und Peptid 96-110,
- Peptid 19-104 und Peptid 17-31,
- Peptid 96-110 und Peptid 90-104 und
- Peptide 93-107 und 128-142 und wenigstens eins der Peptide 17-31, 19-33, 96-110 oder 90-104,
ausgewählt sind.

21. Immunogene Zusammensetzung gemäß einem der Ansprüche 17 bis 20, **dadurch gekennzeichnet, dass** sie ein universelles CD4+T-Epitop umfasst.

22. *In-vitro*-Verfahren zur Diagnose, Evaluierung der Prognose oder der Nachbeobachtung der Behandlung eines Krebses bei einem Individuum, **dadurch gekennzeichnet, dass** es umfasst:
- *Inkontaktbringen, in vitro,* einer biologischen Probe des Individuums mit einem Peptid gemäß einem der Ansprüche 1 bis 8 und
- die Detektion von CD4+-T-Lymphozyten, die für Survivin spezifisch sind, durch jedes geeignete Mittel.

23. Verfahren zur Sortierung von CD4+-T-Lymphozyten, die spezifisch für Survivin sind, **dadurch gekennzeichnet, dass** es wenigstens die folgenden Stufen umfasst:
- *Inkontaktbringen, in vitro,* einer zellulären Probe mit multimeren Komplexen HLA-II/Peptid, die markiert sind, die durch Bindung von löslichen HLA-II-Molekülen mit wenigstens einem Peptid gemäß einem der Ansprüche 1 bis 8 gebildet wurden und
- Sortierung der Zellen, die an die HLA-II/Peptid-Komplexe gebunden sind.

24. Peptid, **dadurch gekennzeichnet, dass** es sich um ein Peptid, wie es gemäß einem der Ansprüche 1 bis 8 definiert ist, handelt, mit Ausschluss des Peptids, das sich zwischen den Positionen 89 bis 101 der Isoform alpha des Survivins befindet.

25. Mehrepitopenfragement, umfassend die Verkettung von wenigstens zwei gleichen oder verschiedenen Epitopen, worin wenigstens ein CD4+T-Epitop des Survivins, enthalten in einem Peptid, wie es in einem der Ansprüche 1 bis 8 definiert ist, und wenigstens ein CD8+-T-Epitop des Survivins und/oder ein universelles CD4+T-Epitop ist.

26. Fusionsprotein, bestehend aus einem Protein oder einem Proteinfragment, fusioniert an ein Peptid, wie es in einem der Ansprüche 1 bis 8 definiert ist, oder ein Mehrepitopenfragment gemäß Anspruch 25.

27. Lipopeptid, erhalten durch Addition eines Lipids an eine α-Aminofunktion oder eine reaktive Funktion der Seitenkette eines Peptids, wie es in einem der Ansprüche 1 bis 8 definiert ist, oder eines Mehrepitopenfragments gemäß Anspruch 25.

28. Polynucleotid, das für ein Peptid gemäß Anspruch 24, ein Mehrepitopenfragment gemäß Anspruch 25 oder ein Fusionsprotein gemäß Anspruch 26 codiert.

29. Expressionsvektor, umfassend ein Polynucleotid gemäß Anspruch 28 unter der Kontrolle geeigneter regulatorischer Sequenzen der Transkription und gegebenenfalls der Translation.

30. Wirtszelle, die durch ein Polynucleotid gemäß Anspruch 28 oder einen Expressionsvektor gemäß Anspruch 29 modifiziert ist.

31. Diagnosekit zur Diagnose, Evaluierung der Prognose oder der Nachbeobachtung der Behandlung von Krebs, **dadurch gekennzeichnet, dass** er ein Peptid gemäß Anspruch 24 umfasst.

## Claims

1. Peptide derived from the alpha-isoform of survivin, for use as an antigen in the prophylactic or therapeutic immunization against cancer, said peptide being selected from the group consisting of:
a) the peptides of 13 to 18 consecutive amino acids located between positions 17 and 34 of the alpha-isoform of survivin,
b) the peptides of 13 to 30 consecutive amino acids located between positions 84 and 113 of the alpha-isoform of survivin, and
c) the peptides of 13 to 21 consecutive amino acids located between positions 122 and 142 of the alpha-isoform of survivin,
which peptides in a), b) and c) bind with a binding activity of less than 1000 nM to at least four HLA II molecules predominant in the Caucasian population, selected from the group consisting of HLA-DR1, HLA-DR3, HLA-DR4, HLA-DR7, HLA-DR11, HLA-DR13, HLA-DR15, HLA-DRB3, HLA-DRB4, HLA-DRB5 and HLA-DP4 molecules encoded, respectively, by the HLA alleles DRB1*0101, DRB1*0301, DRB1*0401, DRB1*0701, DRB1*1101, DRB1*1301, DRB1*1501, DRB3*0101, DRB4*0101, DRB5*0101, DP*0401 and DP*0402, the binding activity corresponding to the concentration of said peptide which inhibits 50% of the maximum binding of a biotynated tracer in a competitive HLA II molecule/peptide binding assay carried out under the conditions as specified in the table below for each HLA II allele:
| Alleles | Tracers (SEQ ID No.:) | Tracer concentration (nM) | Optimum pH | Incubation time (h) |
|---|---|---|---|---|
| DRB1*0101 | 29 | 1 | 6 | 24 |
| DRB1*0301 | 31 | 200 | 4.5 | 72 |
| DRB1*0401 | 29 | 30 | 6 | 24 |
| DRB1*0701 | 33 | 10 | 5 | 24 |
| DRB1*1101 | 29 | 10 | 5 | 24 |
| DRB1*1301 | 32 | 100 | 4.5 | 72 |
| DRB1*1501 | 30 | 30 | 4.5 | 72 |
| DRB5*0101 | 29 | 10 | 5.5 | 24 |
| DRB3*0101 | 34 | 10 | 5.5 | 24 |
| DRB4*0101 | 35 | 10 | 5 | 72 |
| DBP1*0401 | 36 | 1 | 5 | 24 |
| DPB1*0402 | 36 | 1 | 5 | 24 |
and which peptides in a), b) and c) are capable of inducing survivin-specific CD4+ T lymphocytes.

2. Peptide for use as an antigen in the prophylactic or therapeutic immunization against cancer according to Claim 1, **characterized in that** said peptide is capable of being presented by at least one HLA-DP401 or HLA-DP402 molecule.

3. Peptide for use as an antigen in the prophylactic or therapeutic immunization against cancer according to Claim 1 or 2, **characterized in that** it is selected from the group consisting of the peptides: 17-31, 19-33, 20-34, 84-98, 90-104, 91-105, 93-107, 96-110 and 128-142.

4. Peptide for use as an antigen in the prophylactic or therapeutic immunization against cancer according to Claim 3, **characterized in that** said peptide is capable of being presented by an HLA-DP401 or HLA-DP402 molecule, and **in that** it is selected from the group consisting of the peptides: 17-31, 19-33, 20-34, 84-98, 90-104, 91-105, 93-107 and 96-110.

5. Peptide for use as an antigen in the prophylactic or therapeutic immunization against cancer according to Claim 3 or 4, **characterized in that** it is selected from the group consisting of the sequences SEQ ID Nos: 5, 6, 7, 17, 19, 20, 21, 23 and 28.

6. Peptide for use as an antigen in the prophylactic or therapeutic immunization against cancer according to any one of Claims 1 to 5, **characterized in that** said peptide has a sequence of 13, 14, 15, 16, 17, 18, 19, 20, 21, 22, 23, 24 or 25 amino acids.

7. Peptide for use as an antigen in the prophylactic or therapeutic immunization against cancer according to any one of Claims 1 to 6, **characterized in that** said peptide is labeled or complexed.

8. Peptide for use as an antigen in the prophylactic or therapeutic immunization against cancer according to Claim 7, **characterized in that** said peptide is complexed with a labeled HLA II molecule.

9. Polyepitopic fragment comprising the concatenation of at least two identical or different epitopes, at least one of which is a survivin CD4+ T epitope included in a peptide as defined in any one of Claims 1 to 6, for use as an antigen in the prophylactic or therapeutic immunization against cancer.

10. Polyepitopic fragment for use as an antigen in the prophylactic or therapeutic immunization against cancer according to Claim 9, **characterized in that** said polyepitopic fragment comprises the concatenation of at least one survivin CD4+ T epitope included in a peptide as defined in any one of Claims 1 to 6 and of at least one survivin CD8+ T epitope and/or one universal CD4+ T epitope.

11. Polyepitopic fragment for use as an antigen according to Claim 10, **characterized in that** the CD8+ T epitope is chosen from the sequences SEQ ID Nos: 37 to 39.

12. Fusion protein consisting of a protein or a protein fragment, fused to a peptide as defined in any one of Claims 1 to 6 or a polyepitopic fragment as defined in any one of Claims 9 to 11, for use as an antigen in the prophylactic or therapeutic immunization against cancer.

13. Fusion protein for use as an antigen in the prophylactic or therapeutic immunization against cancer according to Claim 12, **characterized in that** said protein or said fragment is selected from the group consisting of an alpha- or beta-chain of an HLA II molecule, a fragment of said chain corresponding to a soluble HLA II molecule and a sequence for targeting to the endosome.

14. Fusion protein for use as an antigen in the prophylactic or therapeutic immunization against cancer according to Claim 13, **characterized in that** said targeting sequence is derived from the invariable chain Ii or from the LAMP-1 protein.

15. Lipopeptide obtained by addition of a lipid to an α-amino function or a reactive function of the side chain of a peptide as defined in any one of Claims 1 to 6 or of a polyepitopic fragment as defined in any one of Claims 9 to 11, for use as an antigen in the prophylactic or therapeutic immunization against cancer.

16. Expression vector comprising a polynucleotide encoding a peptide as defined in any one of Claims 1 to 6, a polyepitopic fragment as defined in any one of Claims 9 to 11 or a fusion protein as defined in any one of Claims 12 to 14, under the control of appropriate regulatory sequences, for use as an antigen in the prophylactic or therapeutic immunization against cancer.

17. Immunogenic or vaccinal composition, **characterized in that** it comprises at least one peptide according to any one of Claims 1 to 8, one polyepitopic fragment according to Claim 10 or 11, one fusion protein according to any one of Claims 12 to 14, one lipopeptide according to Claim 15 or one vector according to Claim 16, and a pharmaceutically acceptable carrier, a carrier substance or an adjuvant.

18. Immunogenic or vaccinal composition according to Claim 17, **characterized in that** it comprises at least one peptide according to any one of Claims 1 to 8 and one peptide including a survivin CD8+ T epitope, in the form of a mixture of peptides, of a polyepitopic fragment and/or of an expression vector encoding said peptides or said fragment.

19. Immunogenic or vaccinal composition according to Claim 18, **characterized in that** said CD8+ T epitope is chosen from the sequences SEQ ID Nos: 37 to 39.

20. Immunogenic or vaccinal composition according to any one of Claims 17 to 19, **characterized in that** it comprises at least two different peptides as defined in any one of Claims 1 to 8, selected from the group consisting of one of the following combinations:
- peptide 17-31 and at least one of peptides 19-33, 90-104 or 128-142,
- peptide 19-33 and peptide 96-110,
- peptide 90-104 and peptide 17-31,
- peptide 96-110 and peptide 90-104, and
- peptides 93-107 and 128-142, and at least one of peptides 17-31, 19-33, 96-110 or 90-104.

21. Immunogenic composition according to any one of Claims 17 to 20, **characterized in that** it comprises a universal CD4+ T epitope.

22. *In vitro* method for diagnosing, evaluating the prognosis of or monitoring the treatment of a cancer in an individual, **characterized in that** it comprises:
- bringing a biological sample from said individual into contact, *in vitro,* with a peptide according to any one of Claims 1 to 8, and
- detecting survivin-specific CD4+ T lymphocytes by any appropriate means.

23. Method for sorting survivin-specific CD4+ T lymphocytes, **characterized in that** it comprises at least the following steps:
- bringing a cell sample into contact, *in vitro,* with labeled HLA II/peptide multimeric complexes formed by the binding of soluble HLA II molecules with at least one peptide according to any one of Claims 1 to 8, and
- sorting the cells bound to said HLA II/peptide complexes.

24. Peptide **characterized in that** it is a peptide as defined in any one of Claims 1 to 8, with the exclusion of the peptide located between positions 89 and 101 of the alpha-isoform of survivin.

25. Polyepitopic fragment comprising the concatenation of at least two identical or different epitopes, including at least one survivin CD4+ T epitope included in a peptide as defined in any one of Claims 1 to 8 and at least one survivin CD8+ epitope and/or one universal CD4+ T epitope.

26. Fusion protein consisting of a protein or a protein fragment, fused to a peptide as defined in any one of Claims 1 to 8 or a polyepitopic fragment according to Claim 25.

27. Lipopeptide obtained by the addition of a lipid to an α-amino function or a reactive function of the side chain of a peptide as defined in any one of Claims 1 to 8 or of a polyepitopic fragment according to Claim 25.

28. Polynucleotide encoding a peptide according to Claim 24, a polyepitopic fragment according to Claim 25 or a fusion protein according to Claim 26.

29. Expression vector comprising a polynucleotide according to Claim 28 under the control of appropriate regulatory sequences for transcription and, optionally, for translation.

30. Host cell modified with a polynucleotide according to Claim 28 or an expression vector according to Claim 29.

31. Kit for diagnosing, for evaluating the prognosis of or for monitoring the treatment of cancer, **characterized in that** it comprises a peptide according to Claim 24.
